# EUROPEAN PATENT APPLICATION

(11) **EP 4 190 803 A1**
(43) Date of publication of application: **07.06.2023**
(21) Application number: 21848660.3
(22) Date of filing: 30.07.2021
(51) Int. Cl.: C07K 14/47, C07K 14/705, A61K 38/17

(54) **SIRPa-FC FUSION PROTEIN**

(30) Priority: 30.07.2020 CN 202010748225
(71) Applicant: Sunshine Guojian Pharmaceutical (Shanghai) Co., Ltd., China (Shanghai) Pilot Free Trade Zone Shanghai 201203 (CN)
(72) Inventor: ZHAO, Jie, Shanghai 201203 (CN); HUANG, Haomin, Shanghai 201203 (CN); ZHU, Zhenping, Shanghai 201203 (CN)
(74) Representative: reuteler & cie SA
(86) International application number: PCT/CN2021/109824
(87) International publication number: WO 2022/022709

(57) **Abstract**

The present invention relates to the technical field of fusion proteins, and particularly to an SIRPα-Fc fusion protein. The fusion protein comprises an SIRPα D1 domain variant and an immunoglobulin Fc region. The fusion protein of the present invention has the potential to treat diseases associated with the SIRPα-CD47 signaling pathway.

## Description

### TECHNICAL FIELD

The present invention relates to a fusion protein, and particularly to a SIRPα-Fc fusion protein.

### BACKGROUND

Phagocytosis is a multi-step cellular process involving target cell recognition, phagocytosis and lysosomal digestion, regulated by receptor-ligand (i.e., checkpoint) interactions between target cells and phagocytes (such cells play an important role in immunosurveillance on tumor cells). Healthy normal tissues and cells inherently have the ability to express antiphagocytic molecules to avoid being cleared by phagocytic cells. However, cancer cells also have learned a similar mechanism and rely more on it than normal cells. Based on the knowledge of the way cancer cells evade immune attack, scientists believe that targeting the checkpoints that regulate phagocytosis (simply referred to as phagocytosis checkpoints, such as CD47-SIRPα) may provide a new approach to the development of cancer immunotherapy.

CD47-targeting monoclonal antibodies can clear tumor cells by strengthening macrophages' phagocytosis. The related monoclonal antibody drugs are in the stage of clinical development. However, clinical results also show that CD47 monoclonal antibodies have some significant toxic and side effects; for example, the use of CD47 monoclonal antibodies can cause anemia in patients as human red cells also highly express CD47. Although in April 2019, the therapy using soluble SIRPα-Fc fusion protein TTI-621 to block the CD47-SIRPα axis showed promising early clinical outcomes in patients with Sézary syndrome (a variant of cutaneous T cell lymphoma) (reference: Johnson L D, Banerjee S, Kruglov O, et al. Targeting CD47 in Sézary syndrome with SIRPαFc[J]. Blood Advances, 2019, 3(7): 1145-1153), the current SIRPα-Fc fusion proteins still have limitations in terms of target binding activity and the like.

Therefore, there is an urgent need in the art to develop SIRPα-Fc fusion proteins with high affinity and low toxicity and side effect.

### SUMMARY

The present invention is intended to provide a high-affinity SIRPα-Fc fusion protein comprising an SIRPα D1 domain variant and an immunoglobulin Fc region. The fusion protein can bind to ligand CD47 with high affinity in one aspect and retains the Fc's binding activity for Fc receptors in another aspect. The present invention is also intended to provide a nucleic acid molecule encoding the fusion protein, to provide an expression vector comprising the nucleic acid molecule, to provide a host cell comprising the expression vector, to provide a method for preparing the fusion protein, to provide a pharmaceutical composition comprising the fusion protein, and to provide use of the fusion protein or the pharmaceutical composition for the preparation of a medicament for the treatment of a tumor.

In order to achieve the above purposes, the present invention provides the following technical solutions.

A first aspect of the present invention provides a SIRPα-Fc fusion protein, wherein the fusion protein comprises a SIRPα D1 domain variant and an immunoglobulin Fc region; the SIRPα D1 domain variant comprises, relative to SEQ ID NO: 1, at least one set of mutations selected from the group consisting of H24N; V27L; I31L; E47Q; E70S; I81V; A84E; R114L; E2T and E1 deletions; L4V and Q5K; A21T and I22V; E65D and S66A; and F103I and K104Q.

In one preferred embodiment, the SIRPα D1 domain variant comprises, relative to SEQ ID NO: 1, I31L and at least one additional set of mutations selected from the group consisting of H24N; V27L; E47Q; E70S; I81V; A84E; R114L; E2T and E1 deletions; L4V and Q5K; A21T and I22V; E65D and S66A; and F103I and K104Q.

In one preferred embodiment, the SIRPα D1 domain variant comprises, relative to SEQ ID NO: 1, at least two sets of mutations.

In one preferred embodiment, the SIRPα D1 domain variant comprises, relative to SEQ ID NO: 1, the following two sets of mutations: V27L; and I31L.

In one preferred embodiment, the SIRPα D1 domain variant comprises, relative to SEQ ID NO: 1, at least three sets of mutations.

In one preferred embodiment, the SIRPα D1 domain variant comprises, relative to SEQ ID NO: 1, at least the following three sets of mutations: V27L; I31L; and L4V and Q5K.

In one preferred embodiment, the SIRPα D1 domain variant comprises, relative to SEQ ID NO: 1, at least four sets of mutations.

In one preferred embodiment, the SIRPα D1 domain variant comprises, relative to SEQ ID NO: 1, at least the following four sets of mutations: V27L; I31L; L4V and Q5K; and E2T and E1 deletions.

In one preferred embodiment, the SIRPα D1 domain variant comprises, relative to SEQ ID NO: 1, at least five sets of mutations.

In one preferred embodiment, the SIRPα D1 domain variant comprises, relative to SEQ ID NO: 1, at least the following five sets of mutations: V27L; I31L; L4V and Q5K; E2T and E1 deletions; and E65D and S66A;
or the SIRPα D1 domain variant comprises, relative to SEQ ID NO: 1, the following five sets of mutations: V27L; I31L; E65D and S66A; E47Q; and E70S.

In one preferred embodiment, the SIRPα D1 domain variant comprises, relative to SEQ ID NO: 1, at least six sets of mutations.

In one preferred embodiment, the SIRPα D1 domain variant comprises, relative to SEQ ID NO: 1, at least the following six sets of mutations: V27L; I31L; L4V and Q5K; E2T and E1 deletions; E65D and S66A; and E47Q.

In one preferred embodiment, the SIRPα D1 domain variant comprises, relative to SEQ ID NO: 1, at least seven sets of mutations.

In one preferred embodiment, the SIRPα D1 domain variant comprises, relative to SEQ ID NO: 1, at least the following seven sets of mutations: V27L; I31L; L4V and Q5K; E2T and E1 deletions; E65D and S66A; E47Q; and E70S.

In another preferred example, the SIRPα D1 domain variant further comprises, relative to SEQ ID NO: 1, at least one set of mutations at positions selected from the group consisting of V6; K53; H56; and A66; preferably, two sets of mutations at positions V6 and H56; or three sets of mutations at positions V6; H56; and A66; or four sets of mutations at positions: V6; H56; A66; and K53.

In another preferred example, the mutations are V6L; K53R; H56N; H56R; H56Q; A66L; and A66T or A66G.

In another preferred example, the SIRPα D1 domain variant comprises, relative to SEQ ID NO: 1, the following six sets of mutations: V6L; V27L; I31L; E65D and S66A; E47Q; and E70S; or the following seven sets of mutations: V6L; V27L; I31L; E65D and S66A; E47Q; E70S; and H56N; or the following seven sets of mutations: V6L; V27L; I31L; E65D and S66A; E47Q; E70S; and H56R; or the following eight sets of mutations: V6L; V27L; I31L; E65D and S66A; E47Q; E70S; H56R; and A66G; or the following nine sets of mutations: V6L; V27L; I31L; E65D and S66A; E47Q; E70S; H56R; A66G; and K53R.

In one preferred embodiment, the SIRPα D1 domain variant has an amino acid sequence selected from the group consisting of SEQ ID NO: 32 to SEQ ID NO: 39.

In another preferred embodiment, the SIRPα D1 domain variant has an amino acid sequence selected from the group consisting of SEQ ID NO: 47 to SEQ ID NO: 51.

In one preferred embodiment, the immunoglobulin Fc region is selected from the group consisting of human IgG1-Fc region, human IgG2-Fc region, human IgG3-Fc region and human IgG4-Fc region.

In one preferred embodiment, the immunoglobulin Fc region comprises CH2 and CH3 domains, and preferably further comprises a hinge region between heavy chain CH1 and CH2 domains.

In one preferred embodiment, the human IgG1-Fc region comprises an amino acid sequence set forth in SEQ ID NO: 2.

In another preferred embodiment, the human IgG4-Fc region comprises an amino acid sequence set forth in SEQ ID NO: 40.

In one preferred embodiment, the SIRPα D1 domain variant is linked to the N-terminus or C-terminus of the immunoglobulin Fc region by a peptide linker.

In one preferred embodiment, the peptide linker comprises an amino acid sequence set forth in SEQ ID NO: 3.

Those skilled in the art can select a peptide linker depending on the flexibility they want the fusion protein to have. Other optional peptide linker forms include G, GS, SG, GGS, GSG, SGG, GGG, GGGS, SGGG, GGGGSGS, GGGGSGGS, GGGGSGGGGS, GGGGSGGGGSGGGGS, AKTTPKLEEGEFSEAR, AKTTPKLEEGEFSEARV, AKTTPKLGG, SAKTTPKLGG, SAKTTP, RADAAP, RADAAPTVS, RADAAAAGGPGS, SAKTTPKLEEGEFSEARV, ADAAP, ADAAPTVSIFPP,
TVAAP, TVAAPSVFIFPP, QPKAAP, QPKAAPSVTLFPP, AKTTPP, AKTTPPSVTPLAP, AKTTAPSVYPLAP, ASTKGP, ASTKGPSVFPLAP, GENKVEYAPALMALS,

GPAKELTPLKEAKVS, GHEAAAVMQVQYPAS, and the like.

In one preferred embodiment, the fusion protein has an amino acid sequence selected from the group consisting of SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 41, SEQ ID NO: 42, SEQ ID NO: 43, SEQ ID NO: 44 and SEQ ID NO: 45.

A second aspect of the present invention provides a nucleic acid molecule, wherein the nucleic acid molecule encodes the fusion protein.

In one preferred embodiment, the nucleic acid molecule has a nucleic acid sequence selected from the group consisting of SEQ ID NO: 16, SEQ ID NO: 18, SEQ ID NO: 20, SEQ ID NO: 22, SEQ ID NO: 24, SEQ ID NO: 26, SEQ ID NO: 28 and SEQ ID NO: 30.

It is known to those skilled in the art that proper substitutions, deletions, alterations or insertions can be introduced into the nucleic acid molecule encoding the amino acid sequence of the fusion protein described above to afford a homolog of the nucleic acid molecule.

A third aspect of the present invention provides an expression vector, wherein the expression vector comprises the nucleic acid molecule described above.

A fourth aspect of the present invention provides a host cell, wherein the host cell comprises the expression vector described above.

A fifth aspect of the present invention provides a method for preparing a fusion protein, wherein the method comprises the following steps:
a) culturing the host cell described above under expression conditions so the SIRPα-Fc fusion protein is expressed; and
b) isolating and purifying the fusion protein described in step a).A sixth aspect of the present invention provides a pharmaceutical composition, wherein the pharmaceutical composition comprises an effective amount of the fusion protein described above and one or more pharmaceutically acceptable carriers, diluents or excipients.

A seventh aspect of the present invention provides use of the fusion protein and the pharmaceutical composition described above for the preparation of a medicament for the treatment of a tumor, wherein tumor cells of the tumor express CD47.

According to the present invention, the tumor is selected from the group consisting of melanoma, kidney cancer, prostate cancer, pancreatic cancer, breast cancer, colon cancer, lung cancer, oesophageal cancer, head and neck squamous cell carcinoma, liver cancer, ovarian cancer, cervical cancer, thyroid cancer, glioblastoma, neuroglioma, leukaemia, lymphoma, myeloma and gastric cancer. Preferably, the melanoma is metastatic malignant melanoma; the kidney cancer is clear cell renal cell carcinoma; the prostate cancer is hormone-refractory prostate adenocarcinoma; the lung cancer is non-small cell lung cancer.

An eighth aspect of the present invention provides a method for treating a tumor, which comprises administering to a subject the fusion protein and the pharmaceutical composition described above, wherein tumor cells of the tumor express CD47.

In another preferred example, the tumor is selected from the group consisting of melanoma, kidney cancer, prostate cancer, pancreatic cancer, breast cancer, colon cancer, lung cancer, oesophageal cancer, head and neck squamous cell carcinoma, liver cancer, ovarian cancer, cervical cancer, thyroid cancer, glioblastoma, neuroglioma, leukaemia, lymphoma, myeloma and gastric cancer.

A ninth aspect of the present invention provides an immunoconjugate, wherein the immunoconjugate comprises:
(a) the fusion protein described above; and
(b) a conjugated moiety selected from the group consisting of a detectable label, a drug, a toxin, a cytokine, a radionuclide and an enzyme.

In another preferred example, the immunoconjugate is used for the preparation of a pharmaceutical composition for the treatment of a tumor.

A tenth aspect of the present invention provides an SIRPα-Fc mutant protein, wherein the mutant protein comprises an SIRPα D1 protein domain variant; the SIRPα D1 domain variant comprises, relative to SEQ ID NO: 1, at least one or more sets of mutations selected from the group consisting of H24N; V27L; I31L; E47Q; E70S; I81V; A84E; R114L; E2T and E1 deletions; L4V and Q5K; A21T and I22V; E65D and S66A; F103I and K104Q; V6L; K53R; H56N; H56R; H56Q; A66L; and A66T or A66G.

In another preferred example, the domain variant comprises, relative to SEQ ID NO: 1, the following six sets of mutations: V6L; V27L; I31L; E65D and S66A; E47Q; and E70S; or the following seven sets of mutations: V6L; V27L; I31L; E65D and S66A; E47Q; E70S; and H56N; or the following seven sets of mutations: V6L; V27L; I31L; E65D and S66A; E47Q; E70S; and H56R; or the following eight sets of mutations: V6L; V27L; I31L; E65D and S66A; E47Q; E70S; H56R; and A66G; or the following nine sets of mutations: V6L; V27L; I31L; E65D and S66A; E47Q; E70S; H56R; A66G; and K53R.

In another preferred example, the SIRPα mutant protein is a mutant SIRPα D1 protein domain variant.

An eleventh aspect of the present invention provides a nucleic acid molecule, wherein the nucleic acid molecule encodes the mutant protein according to the tenth aspect of the present invention or a fusion protein thereof.

A twelfth aspect of the present invention provides an expression vector, wherein the expression vector comprises the nucleic acid molecule according to the eleventh aspect of the present invention.

A thirteenth aspect of the present invention provides a host cell, wherein the host cell comprises the expression vector according to the twelfth aspect of the present invention.

A fourteenth aspect of the present invention provides a pharmaceutical composition, wherein the pharmaceutical composition comprises: (i) an effective amount of the mutant protein according to the tenth aspect of the present invention or a fusion protein thereof; and (ii) one or more pharmaceutically acceptable carriers, diluents or excipients.

A fifteenth aspect of the present invention provides a fusion protein, wherein the fusion protein comprises (a) a first protein element, wherein the first protein element is the mutant protein according to the tenth aspect of the present invention, and (b) a second protein element fused with element (a), wherein the second protein element is not derived from SIRPα protein.

In another preferred example, the second protein element includes a protein elements for prolonging the half-life, or a protein element for providing another activity.

The advantageous effects of the present invention: An SIRPα-Fc fusion protein comprising an SIRPα D1 domain variant and an immunoglobulin Fc region is constructed in the present invention. The fusion protein can bind to CD47 with high affinity; it has significantly higher relative affinity for ligand CD47 than the wild type at the protein level. Further, the relative affinity of the SIRPα-Fc fusion protein for ligand CD47 is further improved by combining the mutation modes of the SIRPα D1 domain variant of the present invention. The high-affinity SIRPα-Fc fusion protein of the present invention can efficiently block the binding of SIRPα to CD47 and has lower toxic and side effects, thereby having the potential to treat diseases associated with the SIRPα-CD47 signaling pathway.

### BRIEF DESCRIPTION OF THE DRAWINGS

FIG. 1: ELISA assay for the relative affinity of SIRP-Fc-IgG1, mSIRP-Fc-IgG1, magrolimab-IgG1 and anti-CD47B-Hu-IgG1 for CD47.
FIG. 2: alignment of amino acid sequences of human and NOD mouse SIRPα D1 domains.
FIG. 3: ELISA assay for the relative affinity of SIRP-Fc-IgG1 mutants for CD47.
FIG. 4: flow cytometry assay for the binding capacity of SIRP-Fc-IgG1 mutants comprising mutation combinations to Daudi cells.
FIG. 5: ELISA assay for the relative affinity of mutants of SIRP6 series for CD47.
FIG. 6: ELISA assay for the relative affinity of SIRP6 mutants comprising mutation combinations (V6L+H56N, and V6L+H56R) for CD47.
FIG. 7: ELISA assay for the relative affinity of SIRP6 mutants comprising mutation combinations (V6L+H56R+A66G, and V6L+H56R) for CD47.
FIG. 8: ELISA assay for the relative affinity of SIRP6 mutants comprising mutation combinations (V6L+H56R+A66G, V6L+H56R+A66G+K53R) for CD47.
FIG. 9: assay for the ADCC activity of SIRP mutants.
FIG. 10: assay for the ADCP activity of SIRP mutants, with Daudi cells as target cells.
FIG. 11: assay for the ADCP activity of SIRP mutants, with Jurkat cells as target cells.
FIG. 12: antitumor effects of mutants in mice.
FIG. 13: FIG. A shows the results of treatment of red cells with SIRP6-IgG1;
FIG. B shows the results of treatment of red cells with anti-CD47B-Hu-IgG1.

### DETAILED DESCRIPTION

The inventor obtains a series of SIRPα-Fc fusion proteins capable of binding to CD47 with high affinity and mutants thereof through extensive and in-depth research and massive screening. The high-affinity SIRPα-Fc fusion proteins of the present invention can efficiently block the binding of SIRP to CD47, and particularly, the SIRP10-Fc fusion protein has the highest affinity for CD47. In addition, the SIRPα-Fc fusion proteins of the present invention have low toxicity and side effects while having high blocking activity, and therefore have better medication window. The present invention is achieved on this basis.

### CD47 and SIRPα Proteins

The CD47-SIRPα axis is the first discovered and also the most well researched phagocytosis checkpoint (reference: Seiffert M, Cant C, Chen Z, et al. Human signal-regulatory protein is expressed on normal, but not on subsets of leukemic myeloid cells and mediates cellular adhesion involving its counterreceptor CD47. Blood. 1999; 94(11):3633-3643).

The full name of SIRPα is signal regulatory protein α. As the first member of the SIRP family, it was identified in the late 1990s. It is expressed on myeloid cells, including all types of macrophages. All the SIRPs comprise an N-terminal extracellular domain, a single transmembrane domain and a C-terminal intracellular domain. SIRP proteins can be divided into at least three subfamily subtypes, referred to as SIRPα, SIRPβ and SIRPγ, according to the structures of their transmembrane and/or intracellular domains and their potential roles in signal transduction. SIRPα has a longer intracellular domain and comprises two immunoreceptor tyrosine-based inhibitory motifs (ITIMs). SIRPα is identified as an extracellular receptor/ligand for CD47. CD47 is another important member of the cell surface immunoglobulin superfamily. Studies show that the CD47 binding site on SIRPα is located in the IgV domain of the extracellular segment. Numerous studies have demonstrated that CD47 is extensively expressed on the surfaces of normal cells and, by binding to SIRPα on the surfaces of macrophages, releases a "don't eat me" signal to protect healthy cells from being "eaten" by macrophages. Since its discovery, SIRPα-CD47 binding interactions have been shown to play a critical role in a variety of important leukocyte functions (including neutrophil and monocyte migration) (references: Liu Y, Tong Q, Zhou Y, et al. Functional Elements on SIRPα IgV domain Mediate Cell Surface Binding to CD47[J]. Journal of Molecular Biology, 2007, 365(3): 680-693; Murata Y, Saito Y, Kotani T, et al. CD47-signal regulatory protein α signaling system and its application to cancer immunotherapy[J]. Cancer Science, 2018, 109(8): 2349-2357).

### Terminology

In the present invention, the terms "antibody (abbreviated as Ab)" and "immunoglobulin G (abbreviated as IgG)" are heterotetrameric proteins having the same structural feature, which consists of two identical light chains (L) and two identical heavy chains (H). Each light chain is linked to a heavy chain by one covalent disulfide bond, while the number of disulfide bonds between the heavy chains of different immunoglobulin isotypes varies. Each heavy chain and each light chain also have regularly spaced intrachain disulfide bonds. At one end of each heavy chain is a variable region (VH) followed by a constant region. The heavy chain constant region consists of three domains: CH1, CH2 and CH3. Each light chain has a variable region (VL) at one end and a constant region at the other end. The light chain constant region comprises one domain CL. The light chain constant region is paired with the CH1 domain of the heavy chain constant region, and the variable region of the light chain is paired with the variable region of the heavy chain. The constant regions are not directly involved in the binding of the antibody to the antigen, but they exhibit different effector functions, such as being involved in the antibody-dependent cell-mediated cytotoxicity (ADCC). Heavy chain constant regions include IgG1, IgG2, IgG3 and IgG4 subtypes; light chain constant regions include κ (kappa) or λ (lambda). The heavy chains and light chains of an antibody are covalently linked together by disulfide bonds between the CH1 domains of the heavy chains and the CL domains of the light chains. The two heavy chains of an antibody are covalently linked together by an inter-polypeptide disulfide bond formed between the hinge regions.

In the present invention, the term "monoclonal antibody (mAb)" refers to an antibody obtained from a substantially homogeneous population-that is, individual antibodies contained in the population are identical, except for a few naturally occurring mutations that may be present. Monoclonal antibodies are highly specific to single antigen sites. Moreover, different from conventional polyclonal antibody formulations (which generally are mixtures of different antibodies specific to different determinants), each monoclonal antibody is specific to a single determinant on the antigen. In addition to their specificity, monoclonal antibodies have the advantage that they can be synthesized by hybridoma culture without contamination by other immunoglobulins. The modifier "monoclonal" indicates the characteristic of the antibody-the antibody is obtained from a substantially homogeneous antibody population, and shall not be construed as requiring any particular method to produce the antibody.

In the present invention, the terms "Fab" and "Fc" mean that papain can cleave an antibody into two identical Fab fragments and one Fc fragment. The Fab fragments consist of the VH and CH1 domains of the heavy chains and the VL and CL domains of the light chains of the antibody. The Fc fragment, i.e., fragment crystallizable (Fc), consists of the CH2 and CH3 domains of the antibody. The Fc fragment has no antigen-binding activity, and it is the site where the antibody interacts with effector molecules or cells.

In the present invention, the term "variable" means that certain portions of the variable regions of the antibody differ in sequence, resulting in the binding and specificity of various particular antibodies to their particular antigens. However, the variability is not evenly distributed throughout the entire span of the variable regions of the antibody. It is concentrated in three segments called complementarity-determining regions (CDRs) or hypervariable regions in the heavy chain variable region and the light chain variable region. The relatively conserved portions of the variable regions are called framework regions (FRs). The variable regions of natural heavy chains and light chains each comprise four FRs substantially in a β-sheet configuration. The FRs are connected by three CDRs that form a connection loop, and in some cases may form part of a β-sheet structure. The CDRs in each chain lie closely together via the FRs and, together with the CDRs of the other chain, form the antigen-binding site of the antibody (see Kabat et al., NIH Publ. No.91-3242, Vol. I, pages 647-669 (1991)).

As used herein, the term "framework region" (FR) refers to amino acid sequences inserted between CDRs, i.e., refers to those portions of the light chain and heavy chain variable regions of an immunoglobulin that are relatively conserved between different immunoglobulins in a single species. The light chains and heavy chains of an immunoglobulin each have four FRs, and they are designated FR1-L, FR2-L, FR3-L and FR4-L, and FR1-H, FR2-H, FR3-H and FR4-H. Accordingly, the light chain variable domain can be referred to as (FR1-L)-(CDR1-L)-(FR2-L)-(CDR2-L)-(FR3-L)-(CDR3-L)-(FR4-L) and the heavy chain variable domain can thus be expressed as (FR1-H)-(CDR1-H)-(FR2-H)-(CDR2-H)-(FR3-H)- (CDR3-H)-(FR4-H). Preferably, the FRs of the present invention are human antibody FRs or derivatives thereof, and the human antibody FRs or derivatives thereof are substantially identical to the naturally-occurring human antibody FRs-that is, the sequence identity between them reaches 85%, 90%, 95%, 96%, 97%, 98% or 99%. Knowing the amino acid sequences of the CDRs, those skilled in the art can readily determine the framework regions FR1-L, FR2-L, FR3-L and FR4-L and/or FR1-H, FR2-H, FR3-H and FR4-H.

As used herein, the term "human framework region" refers to a framework region that is substantially identical (about 85% or more, specifically 90%, 95%, 97%, 99% or 100%) to the framework region of a naturally-occurring human antibody.

### Fusion Protein

In the present invention, the term "fusion protein" refers to a new polypeptide sequence obtained by fusing two or more identical or different polypeptide sequences. The term "fusion" refers to linking directly by peptide bonds or linking via one or more linkers (peptide linkers). The term "linker (peptide linker)" refers to a short peptide, typically 1-30 amino acids in length, which can link two polypeptide sequences.

The term "linker" as used herein refers to one or more amino acid residues inserted between immunoglobulin domains to provide sufficient mobility for the domains of the light and heavy chains to fold into cross-over dual variable region immunoglobulins. In the present invention, a preferred peptide linker refers to a peptide linker linking the SIRPα D1 domain variant to the N-terminus or C-terminus of the immunoglobulin Fc region. Preferably, the peptide linker is a flexible peptide linker. Examples of suitable linkers include monoglycine (Gly), or serine (Ser) residues, and the identities and sequence of the amino acid residues in the linker may vary with the type of secondary structural element that is desired to be implemented in the linker.

In the present invention, the term "SIRPα D1 domain" refers to an SIRPα membrane distal extracellular IgV-like region, which is at the N-terminus of the full-length wild-type SIRPα and mediates binding to CD47.

In the present invention, the term "variant" refers to a peptide comprising at least one amino acid substitution, deletion or insertion relative to the wild-type or naturally-occurring peptide.

In the present invention, the term "SIRPα-Fc fusion protein" refers to a protein formed by fusing an SIRPα D1 domain with an immunoglobulin Fc region. The SIRPα D1 domain includes variant forms thereof.

In the present invention, the term "immunoglobulin Fc region" includes immunoglobulin complete Fc regions and mutants thereof, and Fc region fragments and mutants thereof.

In the present invention, the fusion proteins of the present invention also include conservative variants thereof, which refer to polypeptides comprising at most 10, preferably at most 8, more preferably at most 5, and most preferably at most 3 amino acid substitutions with amino acids which have similar properties compared to the amino acid sequences of the fusion proteins of the present invention. These conservative variant polypeptides are preferably produced by amino acid substitution according to Table A.

**Table A**

| Original residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gin |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gin; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; He | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Coding Nucleic Acid and Expression Vector

The present invention also provides polynucleotide molecules encoding the fusion proteins of the present invention or fragments thereof. The polynucleotides of the present invention may be in DNA form or RNA form. The DNA form includes cDNA, genomic DNA or artificially synthesized DNA. The DNA may be single-stranded or double-stranded. The DNA may be a coding strand or a non-coding strand.

In the present invention, the term "expression vector" refers to a conventional expression vector in the art comprising suitable regulatory sequences such as promoters, terminators and enhancers and the like. The expression vector may be a virus or a plasmid. The expression vector preferably includes pDR1, pcDNA3.4, pDHFR or pTT5.

The relevant sequence, once obtained, can be replicated in large amount by recombination. This is implemented by cloning the sequence into a vector, transferring into a cell, and then isolating from proliferated host cells based on conventional methods.

The present invention also relates to a vector comprising a suitable DNA sequence described above and a suitable promoter or regulatory sequence. These vectors can be used to transform appropriate host cells, allowing them to express proteins. In the present invention, the term "host cell" refers to any conventional host cell in the art, provided that it can enable the stable replication of the vector and the nucleic acid molecule carried can be effectively expressed. The host cell includes prokaryotic expression cells and eukaryotic expression cells, preferably includes: COS, CHO, NS0, sf9, sf21, DH5α, BL21(DE3), TG1, BL21(DE3), 293E cell or HEK293F cell.

### Pharmaceutical Composition and Use

The present invention also provides a composition. Preferably, the composition is a pharmaceutical composition comprising the antibody described above or an active fragment thereof or a fusion protein thereof and a pharmaceutically acceptable carrier. Generally, these materials can be formulated in a non-toxic, inert and pharmaceutically acceptable aqueous carrier medium, wherein the pH is typically about 4-8, preferably about 5-7, although the pH may vary depending on the properties of the material being formulated and the condition being treated. The formulated pharmaceutical composition can be administered via conventional routes, including (but not limited to) intravenous injection, intravenous drip, subcutaneous injection, topical injection, intramuscular injection, intratumoral injection, intraperitoneal injection (e.g., intraperitoneal), intracranial injection or intracavity injection.

In the present invention, the term "pharmaceutical composition" means that the fusion proteins of the present invention can be combined with pharmaceutically acceptable carriers to form pharmaceutical formulation compositions to achieve more stable therapeutic effects. These preparations can ensure the conformational integrity of the core sequence of amino acids of the fusion proteins disclosed in the present invention while protecting the multifunctional groups of the proteins from degradation (including but not limited to aggregation, deamination or oxidation).

The pharmaceutical composition of the present invention comprises a safe and effective amount (e.g., 0.001-99 wt%, preferably 0.01-90 wt%, and more preferably 0.1-80 wt%) of the fusion protein (or a conjugate thereof) described above in the present invention and a pharmaceutically acceptable carrier or excipient. Such vectors include (but are not limited to): saline, buffer, glucose, water, glycerol, ethanol, and combinations thereof. The pharmaceutical formulation shall match the route of administration. The pharmaceutical composition of the present invention may be prepared in the form of injections, for example, using normal saline or an aqueous solution containing glucose and other adjuvants, by a conventional method. The pharmaceutical composition in the form of an injection or a solution is preferably manufactured under sterile conditions. The amount of the active ingredient administered is a therapeutically effective amount, for example, from about 10 µg/kg body weight to about 50 mg/kg body weight per day. In addition, the fusion proteins of the present invention can also be used with an additional therapeutic agent.

In using the pharmaceutical composition, a safe and effective amount of the fusion protein or an immunoconjugate thereof, typically at least about 10 µg/kg body weight, and in most cases no more than about 50 mg/kg body weight, preferably from about 10 µg/kg body weight to about 10 mg/kg body weight, is administered to a mammal. In determining a specific dose, such factors as the route of administration, the health condition of the patient and the like will also be considered, which are well known to skilled physicians.

The SIRPα-Fc fusion proteins of the present invention have blocking activity and also extraordinarily low toxicity and few side effects.

In the present invention, the term "affinity" or "binding capacity" refers to the strength of a binding interaction between two molecules.

In the present invention, the term "effective amount" refers to an amount or dose that produces a desired effect in a treated subject, including improvement in the condition of the subject, after administration of the pharmaceutical composition of the present invention to the patient.

In the present invention, the position of the amino acid residue in the amino acid mutation is the residue number determined on the basis of the amino acid sequence set forth in SEQ ID NO: 1.

The sequence information referred to in the following examples is summarized in sequence listing Table 1.

**Table 1. Sequence listing**

| SEQ ID NO: | Sequence |
|---|---|
| 1 | Amino acid seauence of SIRPα (CAA71403) D1 domain |
| | EEELQVIQPDKSVSVAAGESAILHCTVTSLIPVGPIQWFRGAGPARELIYNQKEGHF PRVTTVSESTKRENMDFSISISNITPADAGTYYCVKFRKGSPDTEFKSGAGTELSVR AKPS |
| 2 | Amino acid sequence of human IgG1 heavy chain Fc segment |
| | DKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKSL SLSPGK |
| 3 | Amino acid sequence of linker |
| | GGGGS |
| 4 | Nucleic acid sequence of SIRP-Fc-IgG1 |
| | GAGGAGGAGCTGCAGGTCATCCAGCCTGATAAGTCTGTGAGCGTGGCTGCTGG AGAGTCTGCTATTCTGCATTGTACAGTGACATCTCTGATCCCTGTGGACCTATT CAGTGGTTTAGAGGAGCTGGACCTGCTAGAGAGCTGATCTATAATCAGAAGGA GGGCCATTTTCCTAGAGTGACAACAGTGTCTGAGTCTACAAAGAGAGAGAATAT GGATTTTTCTATTTCTATTTCTAATATCACCCCTGCTGATGCTGGCACATACTATTG TGTGAAGTTTAGAAAGGGCTCTCCTGATACAGAGTTTAAGTCTGGAGCTGGAAC CGAGCTGTCTGTGAGAGCTAAGCCTTCTGGCGGAGGTGGAAGCGACAAGACCC ACACATGTCCCCCCTGTCCCGCTCCTGAACTGCTGGGAGGCCCTTCCGTGTTCC TGTTCCCCCCTAAGCCCAAGGACACCCTGATGATTTCCAGGACACCCGAGGTGA CCTGTGTGGTGGTGGACGTCAGCCACGAGGACCCCGAGGTGAAATTCAACTGG TACGTCGATGGCGTGGAGGTGCACAACGCTAAGACCAAGCCCAGGGAGGAGC AGTACAATTCCACCTACAGGGTGGTGTCCGTGCTGACCGTCCTCCATCAGGACT GGCTGAACGGCAAAGAGTATAAGTGCAAGGTGAGCAACAAGGCCCTCCCTGCT CCCATCGAGAAGACCATCAGCAAAGCCAAGGGCCAGCCCAGGGAACCTCAAG TCTATACCCTGCCTCCCAGCAGGGAGGAGATGACCAAGAACCAAGTGAGCCTC ACATGCCTCGTCAAGGGCTTCTATCCTTCCGATATTGCCGTCGAGTGGGAGTCC AACGGACAGCCCGAGAACAACTACAAGACAACACCCCCCGTGCTCGATTCCGA TGGCAGCTTCTTCCTGTACTCCAAGCTGACCGTGGACAAGTCCAGATGGCAAC AAGGCAACGTCTTCAGTTGCAGCGTCATGCATGAGGCCCTCCACAACCACTAC ACCCAGAAGAGCCTCTCCCTGAGCCCTGGAAAG |
| 5 | Amino acid sequence of SIRP-Fc-IgG1 |
| | EEELQVIQPDKSVSVAAGESAILHCTVTSLIPVGPIQWFRGAGPARELIYNQKEGHF PRVTTVSESTKRENMDFSISISNITPADAGTYYCVKFRKGSPDTEFKSGAGTELSVR AKPSGGGGSDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEAL HNHYTQKSLSLSPGK |
| 6 | Amino acid sequence of NOD wild-type mouse SIRPα D1 domain |
| | TEVKIQPEKSVSVAAGDSTVLNCTLTSLLPVGPIRWYRGVGQSRQLIYSFTTEHFP RVTNVSDATKRSNLDFSIRISNVTPEDAGTYYCVKFQRGSPDTEIQSGGGTEVYVL AK |
| 7 | Nucleic acid sequence of mSIRP-Fc-IgG1 |
| | ACCGAGGTGAAGGTCATCCAGCCCGAGAAGAGCGTGAGCGTGGCCGCCGGGG ATAGCACAGTGCTGAACTGCACCCTGACCAGCCTGCTGCCCGTGGGCCCCATCC GGTGGTACCGGGGCGTGGGCCAGAGCAGGCAGCTGATCTACAGCTTCACAACA GAGCACTTCCCCAGGGTGACAAACGTGAGCGACGCCACAAAGAGGAGCAACC TGGACTTCAGCATCCGGATCAGCAACGTGACACCCGAGGACGCCGGCACATAC TACTGTGTGAAGTTTCAGAGAGGCAGCCCTGATACAGAGATTCAGTCAGGAGG GGGAACAGAGGTGTACGTGTTGGCCAAGGGCGGAGGTGGAAGCGACAAGACC CACACATGTCCCCCCTGTCCCGCTCCTGAACTGCTGGGAGGCCCTTCCGTGTTC CTGTTCCCCCCTAAGCCCAAGGACACCCTGATGATTTCCAGGACACCCGAGGTG ACCTGTGTGGTGGTGGACGTCAGCCACGAGGACCCCGAGGTGAAATTCAACTG GTACGTCGATGGCGTGGAGGTGCACAACGCTAAGACCAAGCCCAGGGAGGAG CAGTACAATTCCACCTACAGGGTGGTGTCCGTGCTGACCGTCCTCCATCAGGAC |
| | TGGCTGAACGGCAAAGAGTATAAGTGCAAGGTGAGCAACAAGGCCCTCCCTGC TCCCATCGAGAAGACCATCAGCAAAGCCAAGGGCCAGCCCAGGGAACCTCAA GTCTATACCCTGCCTCCCAGCAGGGAGGAGATGACCAAGAACCAAGTGAGCCT CACATGCCTCGTCAAGGGCTTCTATCCTTCCGATATTGCCGTCGAGTGGGAGTCC AACGGACAGCCCGAGAACAACTACAAGACAACACCCCCCGTGCTCGATTCCGA TGGCAGCTTCTTCCTGTACTCCAAGCTGACCGTGGACAAGTCCAGATGGCAAC AAGGCAACGTCTTCAGTTGCAGCGTCATGCATGAGGCCCTCCACAACCACTAC ACCCAGAAGAGCCTCTCCCTGAGCCCTGGAAAG |
| 8 | Amino acid sequence of mSIRP-Fc-IgG1 |
| | TEVKVIQPEKSVSVAAGDSTVLNCTLTSLLPVGPIRWYRGVGQSRQLIYSFTTEHFP RVTNVSDATKRSNLDFSIRISNVTPEDAGTYYCVKFQRGSPDTEIQSGGGTEVYVL AKGGGGSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK |
| 9 | Amino acid sequence of N-terminal IgV-like domain of CD47 extracellular segment |
| | QLLFNKTKSVEFTFCNDTVVIPCFVTNMEAQNTTEVYVKWKFKGRDIYTFDGALN KSTVPTDFSSAKIEVSQLLKGDASLKMDKSDAVSHTGNYTCEVTELTREGETIIELK YRVVSWFSP |
| 10 | Nucleic acid sequence of CD47-His |
| | CAGCTGCTGTTCAACAAGACCAAGAGCGTGGAGTTCACCTTCTGCAACGACAC CGTCGTGATCCCCTGCTTCGTGACCAACATGGAGGCCCAGAACACCACCGAGG TGTACGTGAAGTGGAAGTTCAAGGGCAGAGACATCTACACCTTCGACGGCGCC CTGAACAAGAGCACCGTGCCCACCGATTTTTCTAGCGCCAAGATCGAGGTGTCT CAGCTGCTGAAGGGCGATGCCTCTCTGAAGATGGATAAGTCTGATGCCGTGAGC CACACAGGCAATTATACATGTGAGGTGACAGAGCTGACAAGAGAGGGCGAGAC AATCATCGAGCTGAAGTATAGAGTGGTGTCTTGGTTTAGCCCTGGCGGCGGCGG CAGCCACCACCATCACCACCAC |
| 11 | Amino acid sequence of CD47-His |
| | QLLFNKTKSVEFTFCNDTVVIPCFVTNMEAQNTTEVYVKWKFKGRDIYTFDGALN KSTVPTDFSSAKIEVSQLLKGDASLKMDKSDAVSHTGNYTCEVTELTREGETIIELK YRVVSWFSPGGGGSHHHHHH |
| 12 | Amino acid sequence of heavy chain of positive control antibody magrolimab-IgG1 |
| | QVQLVQSGAEVKKPGASVKVSCKASGYTFTNYNMHWVRQAPGQRLEWMGTIYP GNDDTSYNQKFKDRVTITADTSASTAYMELSSLRSEDTAVYYCARGGYRAMDYW GQGTLVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKSL SLSPGK |
| 13 | Amino acid sequence of light chain of positive control antibody magrolimab-IgG1 |
| | DIVMTQSPLSLPVTPGEPASISCRSSQSIVYSNGNTYLGWYLQKPGQSPQLLIYKVS NRFSGVPDRFSGSGSGTDFTLKISRVEAEDVGVYYCFQGSHVPYTFGQGTKLEIKR TVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 14 | Amino acid sequence of heavy chain of positive control antibody anti-CD47B-Hu-IgG1 |
| | QVQLVQSGAEVKKPGASVKVSCKASGYTFANHVIHWVRQAPGQGLEWMGYIYP YNDGTKYNEKFKDRVTLTSDKSTSTVYMELSSLRSEDTAVYYCARGGYYTYDDW GQGTLVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALP APIEKTISKAKGQPREPQVYTLPPSREEMTKNQVSLTCLVKGFYPSDIAVEWESNGQ PENNYKTTPPVLDSDGSFFLYSKLTVDKSRWQQGNVFSCSVMHEALHNHYTQKSL SLSPGK |
| 15 | Amino acid sequence of light chain of positive control antibody anti-CD47B-Hu-IgG1 |
| | DVVMTQSPLSLPVTLGQPASISCRSSQSLVHSNGKTYLHWYQQRPGQSPRLLIYKV SNRFSGVPDRFSGSGSGTDFTLKISRVEAEDVGVYYCSQSTHVPYTFGQGTKVEIK RTVAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC |
| 16 | Nucleic acid sequence of mutant-I31L fusion protein mutant M8 |
| | Nucleic acid sequence of human IL-22 fusion protein monomer: GAGGAGGAGCTGCAGGTCATCCAGCCTGATAAGTCTGTGAGCGTGGCTGCTGG AGAGTCTGCTATTCTGCATTGTACAGTGACATCTCTGCTGCCTGTGGGACCTATT CAGTGGTTTAGAGGAGCTGGACCTGCTAGAGAGCTGATCTATAATCAGAAGGA GGGCCATTTTCCTAGAGTGACAACAGTGTCTGAGTCTACAAAGAGAGAGAATAT GGATTTTTCTATTTCTATTTCTAATATCACCCCTGCTGATGCTGGCACATACTATTG TGTGAAGTTTAGAAAGGGCTCTCCTGATACAGAGTTTAAGTCTGGAGCTGGAAC CGAGCTGTCTGTGAGAGCTAAGCCTTCTGGCGGAGGTGGAAGCGACAAGACCC ACACATGTCCCCCCTGTCCCGCTCCTGAACTGCTGGGAGGCCCTTCCGTGTTCC TGTTCCCCCCTAAGCCCAAGGACACCCTGATGATTTCCAGGACACCCGAGGTGA CCTGTGTGGTGGTGGACGTCAGCCACGAGGACCCCGAGGTGAAATTCAACTGG TACGTCGATGGCGTGGAGGTGCACAACGCTAAGACCAAGCCCAGGGAGGAGC AGTACAATTCCACCTACAGGGTGGTGTCCGTGCTGACCGTCCTCCATCAGGACT GGCTGAACGGCAAAGAGTATAAGTGCAAGGTGAGCAACAAGGCCCTCCCTGCT CCCATCGAGAAGACCATCAGCAAAGCCAAGGGCCAGCCCAGGGAACCTCAAG TCTATACCCTGCCTCCCAGCAGGGAGGAGATGACCAAGAACCAAGTGAGCCTC ACATGCCTCGTCAAGGGCTTCTATCCTTCCGATATTGCCGTCGAGTGGGAGTCC AACGGACAGCCCGAGAACAACTACAAGACAACACCCCCCGTGCTCGATTCCGA TGGCAGCTTCTTCCTGTACTCCAAGCTGACCGTGGACAAGTCCAGATGGCAAC AAGGCAACGTCTTCAGTTGCAGCGTCATGCATGAGGCCCTCCACAACCACTAC ACCCAGAAGAGCCTCTCCCTGAGCCCTGGAAAG |
| 17 | Amino acid sequence of mutant-I31L fusion protein mutant M8 |
| | EEELQVIQPDKSVSVAAGESAILHCTVTSLLPVGPIQWFRGAGPARELIYNQKEGHF PRVTTVSESTKRENMDFSISISNITPADAGTYYCVKFRKGSPDTEFKSGAGTELSVR AKPSGGGGSDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEAL HNHYTQKSLSLSPGK |
| 18 | Nucleic acid sequence of mutant-131L+V27L fusion protein mutant M27 |
| | GAGGAGGAGCTGCAGGTCATCCAGCCTGATAAGTCTGTGAGCGTGGCTGCTGG AGAGTCTGCTATTCTGCATTGTACACTGACATCTCTGCTGCCTGTGGACCTATT CAGTGGTTTAGAGGAGCTGGACCTGCTAGAGAGCTGATCTATAATCAGAAGGA GGGCCATTTTCCTAGAGTGACAACAGTGTCTGAGTCTACAAAGAGAGAGAATAT GGATTTTTCTATTTCTATTTCTAATATCACCCCTGCTGATGCTGGCACATACTATTG TGTGAAGTTTAGAAAGGGCTCTCCTGATACAGAGTTTAAGTCTGGAGCTGGAAC CGAGCTGTCTGTGAGAGCTAAGCCTTCTGGCGGAGGTGGAAGCGACAAGACCC ACACATGTCCCCCCTGTCCCGCTCCTGAACTGCTGGGAGGCCCTTCCGTGTTCC TGTTCCCCCCTAAGCCCAAGGACACCCTGATGATTTCCAGGACACCCGAGGTGA CCTGTGTGGTGGTGGACGTCAGCCACGAGGACCCCGAGGTGAAATTCAACTGG TACGTCGATGGCGTGGAGGTGCACAACGCTAAGACCAAGCCCAGGGAGGAGC AGTACAATTCCACCTACAGGGTGGTGTCCGTGCTGACCGTCCTCCATCAGGACT GGCTGAACGGCAAAGAGTATAAGTGCAAGGTGAGCAACAAGGCCCTCCCTGCT CCCATCGAGAAGACCATCAGCAAAGCCAAGGGCCAGCCCAGGGAACCTCAAG TCTATACCCTGCCTCCCAGCAGGGAGGAGATGACCAAGAACCAAGTGAGCCTC ACATGCCTCGTCAAGGGCTTCTATCCTTCCGATATTGCCGTCGAGTGGGAGTCC AACGGACAGCCCGAGAACAACTACAAGACAACACCCCCCGTGCTCGATTCCGA TGGCAGCTTCTTCCTGTACTCCAAGCTGACCGTGGACAAGTCCAGATGGCAAC AAGGCAACGTCTTCAGTTGCAGCGTCATGCATGAGGCCCTCCACAACCACTAC ACCCAGAAGAGCCTCTCCCTGAGCCCTGGAAAG |
| 19 | Amino acid sequence of mutant-131L+V27L fusion protein mutant M27 |
| | EEELQVIQPDKSVSVAAGESAILHCTLTSLLPVGPIQWFRGAGPARELIYNQKEGHF PRVTTVSESTKRENMDFSISISNITPADAGTYYCVKFRKGSPDTEFKSGAGTELSVR AKPSGGGGSDKTHTCPPCPAPELLGGPSVFLFPPKPKDTLMISRTPEVTCVVVDVSH |
| | EDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEAL HNHYTQKSLSLSPGK |
| 20 | Nucleic acid sequence of mutant-131L+V27L+L4V+Q5K fusion protein mutant M28 |
| | GAGGAGGAGGTGAAGGTCATCCAGCCTGATAAGTCTGTGAGCGTGGCTGCTGG AGAGTCTGCTATTCTGCATTGTACACTGACATCTCTGCTGCCTGTGGACCTATT CAGTGGTTTAGAGGAGCTGGACCTGCTAGAGAGCTGATCTATAATCAGAAGGA GGGCCATTTTCCTAGAGTGACAACAGTGTCTGAGTCTACAAAGAGAGAGAATAT GGATTTTTCTATTTCTATTTCTAATATCACCCCTGCTGATGCTGGCACATACTATTG TGTGAAGTTTAGAAAGGGCTCTCCTGATACAGAGTTTAAGTCTGGAGCTGGAAC CGAGCTGTCTGTGAGAGCTAAGCCTTCTGGCGGAGGTGGAAGCGACAAGACCC ACACATGTCCCCCCTGTCCCGCTCCTGAACTGCTGGGAGGCCCTTCCGTGTTCC TGTTCCCCCCTAAGCCCAAGGACACCCTGATGATTTCCAGGACACCCGAGGTGA CCTGTGTGGTGGTGGACGTCAGCCACGAGGACCCCGAGGTGAAATTCAACTGG TACGTCGATGGCGTGGAGGTGCACAACGCTAAGACCAAGCCCAGGGAGGAGC AGTACAATTCCACCTACAGGGTGGTGTCCGTGCTGACCGTCCTCCATCAGGACT GGCTGAACGGCAAAGAGTATAAGTGCAAGGTGAGCAACAAGGCCCTCCCTGCT CCCATCGAGAAGACCATCAGCAAAGCCAAGGGCCAGCCCAGGGAACCTCAAG TCTATACCCTGCCTCCCAGCAGGGAGGAGATGACCAAGAACCAAGTGAGCCTC ACATGCCTCGTCAAGGGCTTCTATCCTTCCGATATTGCCGTCGAGTGGGAGTCC AACGGACAGCCCGAGAACAACTACAAGACAACACCCCCCGTGCTCGATTCCGA TGGCAGCTTCTTCCTGTACTCCAAGCTGACCGTGGACAAGTCCAGATGGCAAC AAGGCAACGTCTTCAGTTGCAGCGTCATGCATGAGGCCCTCCACAACCACTAC ACCCAGAAGAGCCTCTCCCTGAGCCCTGGAAAG |
| 21 | Amino acid sequence of mutant-I31L+V27L+L4V+Q5K fusion protein mutant M28 |
| | EEEVKVIQPDKSVSVAAGESAILHCTLTSLLPVGPIQWFRGAGPARELIYNQKEGHF PRVTTVSESTKRENMDFSISISNITPADAGTYYCVKFRKGSPDTEFKSGAGTELSVR AKPSGGGGSDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEAL HNHYTQKSLSLSPGK |
| 22 | Nucleic acid sequence of mutant-131L+V27L+L4V+Q5K+E1#+E2T fusion protein mutant M29 |
| | ACCGAGGTGAAGGTCATCCAGCCTGATAAGTCTGTGAGCGTGGCTGCTGGAGA GTCTGCTATTCTGCATTGTACACTGACATCTCTGCTGCCTGTGGGACCTATTCAG TGGTTTAGAGGAGCTGGACCTGCTAGAGAGCTGATCTATAATCAGAAGGAGGG CCATTTTCCTAGAGTGACAACAGTGTCTGAGTCTACAAAGAGAGAGAATATGGA TTTTTCTATTTCTATTTCTAATATCACCCCTGCTGATGCTGGCACATACTATTGTGT GAAGTTTAGAAAGGGCTCTCCTGATACAGAGTTTAAGTCTGGAGCTGGAACCG AGCTGTCTGTGAGAGCTAAGCCTTCTGGCGGAGGTGGAAGCGACAAGACCCAC ACATGTCCCCCCTGTCCCGCTCCTGAACTGCTGGGAGGCCCTTCCGTGTTCCTG TTCCCCCCTAAGCCCAAGGACACCCTGATGATTTCCAGGACACCCGAGGTGACC TGTGTGGTGGTGGACGTCAGCCACGAGGACCCCGAGGTGAAATTCAACTGGTA CGTCGATGGCGTGGAGGTGCACAACGCTAAGACCAAGCCCAGGGAGGAGCAG TACAATTCCACCTACAGGGTGGTGTCCGTGCTGACCGTCCTCCATCAGGACTGG CTGAACGGCAAAGAGTATAAGTGCAAGGTGAGCAACAAGGCCCTCCCTGCTCC CATCGAGAAGACCATCAGCAAAGCCAAGGGCCAGCCCAGGGAACCTCAAGTCT ATACCCTGCCTCCCAGCAGGGAGGAGATGACCAAGAACCAAGTGAGCCTCACA TGCCTCGTCAAGGGCTTCTATCCTTCCGATATTGCCGTCGAGTGGGAGTCCAAC GGACAGCCCGAGAACAACTACAAGACAACACCCCCCGTGCTCGATTCCGATGG CAGCTTCTTCCTGTACTCCAAGCTGACCGTGGACAAGTCCAGATGGCAACAAG GCAACGTCTTCAGTTGCAGCGTCATGCATGAGGCCCTCCACAACCACTACACCC AGAAGAGCCTCTCCCTGAGCCCTGGAAAG |
| 23 | Amino acid sequence of mutant-131L+V27L+L4V+Q5K+E1#+E2T fusion protein mutant M29 |
| | TEVKVIQPDKSVSVAAGESAILHCTLTSLLPVGPIQWFRGAGPARELIYNQKEGHFP RVTTVSESTKRENMDFSISISNITPADAGTYYCVKFRKGSPDTEFKSGAGTELSVRA |
| | KPSGGGGSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK |
| 24 | Nucleic acid sequence of mutant-I31L+V27L+L4V+Q5K+E1#+E2T+E65D+S66A fusion protein mutant M30 |
| | ACCGAGGTGAAGGTCATCCAGCCTGATAAGTCTGTGAGCGTGGCTGCTGGAGA GTCTGCTATTCTGCATTGTACACTGACATCTCTGCTGCCTGTGGGACCTATTCAG TGGTTTAGAGGAGCTGGACCTGCTAGAGAGCTGATCTATAATCAGAAGGAGGG CCATTTTCCTAGAGTGACAACAGTGTCTGACGCCACAAAGAGAGAGAATATGG ATTTTTCTATTTCTATTTCTAATATCACCCCTGCTGATGCTGGCACATACTATTGTG TGAAGTTTAGAAAGGGCTCTCCTGATACAGAGTTTAAGTCTGGAGCTGGAACC GAGCTGTCTGTGAGAGCTAAGCCTTCTGGCGGAGGTGGAAGCGACAAGACCCA CACATGTCCCCCCTGTCCCGCTCCTGAACTGCTGGGAGGCCCTTCCGTGTTCCT GTTCCCCCCTAAGCCCAAGGACACCCTGATGATTTCCAGGACACCCGAGGTGA CCTGTGTGGTGGTGGACGTCAGCCACGAGGACCCCGAGGTGAAATTCAACTGG TACGTCGATGGCGTGGAGGTGCACAACGCTAAGACCAAGCCCAGGGAGGAGC AGTACAATTCCACCTACAGGGTGGTGTCCGTGCTGACCGTCCTCCATCAGGACT GGCTGAACGGCAAAGAGTATAAGTGCAAGGTGAGCAACAAGGCCCTCCCTGCT CCCATCGAGAAGACCATCAGCAAAGCCAAGGGCCAGCCCAGGGAACCTCAAG TCTATACCCTGCCTCCCAGCAGGGAGGAGATGACCAAGAACCAAGTGAGCCTC ACATGCCTCGTCAAGGGCTTCTATCCTTCCGATATTGCCGTCGAGTGGGAGTCC AACGGACAGCCCGAGAACAACTACAAGACAACACCCCCCGTGCTCGATTCCGA TGGCAGCTTCTTCCTGTACTCCAAGCTGACCGTGGACAAGTCCAGATGGCAAC AAGGCAACGTCTTCAGTTGCAGCGTCATGCATGAGGCCCTCCACAACCACTAC ACCCAGAAGAGCCTCTCCCTGAGCCCTGGAAAG |
| 25 | Amino acid sequence of mutant-I31L+V27L+L4V+Q5K+E1#+E2T+E65D+S66A fusion protein mutant M30 |
| | TEVKVIQPDKSVSVAAGESAILHCTLTSLLPVGPIQWFRGAGPARELIYNQKEGHFP RVTTVSDATKRENMDFSISISNITPADAGTYYCVKFRKGSPDTEFKSGAGTELSVRA KPSGGGGSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK |
| 26 | Nucleic acid sequence of mutant-I31L+V27L+L4V+Q5K+E1#+E2T+E65D+S66A+E47Q fusion protein mutant M31 |
| | ACCGAGGTGAAGGTCATCCAGCCTGATAAGTCTGTGAGCGTGGCTGCTGGAGA GTCTGCTATTCTGCATTGTACACTGACATCTCTGCTGCCTGTGGGACCTATTCAG TGGTTTAGAGGAGCTGGACCTGCTAGACAGCTGATCTATAATCAGAAGGAGGGC CATTTTCCTAGAGTGACAACAGTGTCTGACGCCACAAAGAGAGAGAATATGGAT TTTTCTATTTCTATTTCTAATATCACCCCTGCTGATGCTGGCACATACTATTGTGTG AAGTTTAGAAAGGGCTCTCCTGATACAGAGTTTAAGTCTGGAGCTGGAACCGA GCTGTCTGTGAGAGCTAAGCCTTCTGGCGGAGGTGGAAGCGACAAGACCCACA CATGTCCCCCCTGTCCCGCTCCTGAACTGCTGGGAGGCCCTTCCGTGTTCCTGT TCCCCCCTAAGCCCAAGGACACCCTGATGATTTCCAGGACACCCGAGGTGACCT GTGTGGTGGTGGACGTCAGCCACGAGGACCCCGAGGTGAAATTCAACTGGTAC GTCGATGGCGTGGAGGTGCACAACGCTAAGACCAAGCCCAGGGAGGAGCAGT ACAATTCCACCTACAGGGTGGTGTCCGTGCTGACCGTCCTCCATCAGGACTGGC TGAACGGCAAAGAGTATAAGTGCAAGGTGAGCAACAAGGCCCTCCCTGCTCCC ATCGAGAAGACCATCAGCAAAGCCAAGGGCCAGCCCAGGGAACCTCAAGTCTA TACCCTGCCTCCCAGCAGGGAGGAGATGACCAAGAACCAAGTGAGCCTCACAT GCCTCGTCAAGGGCTTCTATCCTTCCGATATTGCCGTCGAGTGGGAGTCCAACG GACAGCCCGAGAACAACTACAAGACAACACCCCCCGTGCTCGATTCCGATGGC AGCTTCTTCCTGTACTCCAAGCTGACCGTGGACAAGTCCAGATGGCAACAAGG CAACGTCTTCAGTTGCAGCGTCATGCATGAGGCCCTCCACAACCACTACACCCA GAAGAGCCTCTCCCTGAGCCCTGGAAAG |
| 27 | Amino acid sequence of mutant-I31L+V27L+L4V+Q5K+E1#+E2T+E65D+S66A+E47Q |
| | fusion protein mutant M31 |
| | TEVKVIQPDKSVSVAAGESAILHCTLTSLLPVGPIQWFRGAGPARQLIYNQKEGHFP RVTTVSDATKRENMDFSISISNITPADAGTYYCVKFRKGSPDTEFKSGAGTELSVRA KPSGGGGSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK |
| 28 | Nucleic acid sequence of mutant-I31L+V27L+L4V+Q5K+E1#+E2T+E65D+S66A+E47Q+E70S fusion protein mutant M32 |
| | ACCGAGGTGAAGGTCATCCAGCCTGATAAGTCTGTGAGCGTGGCTGCTGGAGA GTCTGCTATTCTGCATTGTACACTGACATCTCTGCTGCCTGTGGGACCTATTCAG TGGTTTAGAGGAGCTGGACCTGCTAGACAGCTGATCTATAATCAGAAGGAGGGC CATTTTCCTAGAGTGACAACAGTGTCTGACGCCACAAAGAGAAGCAATATGGAT TTTTCTATTTCTATTTCTAATATCACCCCTGCTGATGCTGGCACATACTATTGTGTG AAGTTTAGAAAGGGCTCTCCTGATACAGAGTTTAAGTCTGGAGCTGGAACCGA GCTGTCTGTGAGAGCTAAGCCTTCTGGCGGAGGTGGAAGCGACAAGACCCACA CATGTCCCCCCTGTCCCGCTCCTGAACTGCTGGGAGGCCCTTCCGTGTTCCTGT TCCCCCCTAAGCCCAAGGACACCCTGATGATTTCCAGGACACCCGAGGTGACCT GTGTGGTGGTGGACGTCAGCCACGAGGACCCCGAGGTGAAATTCAACTGGTAC GTCGATGGCGTGGAGGTGCACAACGCTAAGACCAAGCCCAGGGAGGAGCAGT ACAATTCCACCTACAGGGTGGTGTCCGTGCTGACCGTCCTCCATCAGGACTGGC TGAACGGCAAAGAGTATAAGTGCAAGGTGAGCAACAAGGCCCTCCCTGCTCCC ATCGAGAAGACCATCAGCAAAGCCAAGGGCCAGCCCAGGGAACCTCAAGTCTA TACCCTGCCTCCCAGCAGGGAGGAGATGACCAAGAACCAAGTGAGCCTCACAT GCCTCGTCAAGGGCTTCTATCCTTCCGATATTGCCGTCGAGTGGGAGTCCAACG GACAGCCCGAGAACAACTACAAGACAACACCCCCCGTGCTCGATTCCGATGGC AGCTTCTTCCTGTACTCCAAGCTGACCGTGGACAAGTCCAGATGGCAACAAGG CAACGTCTTCAGTTGCAGCGTCATGCATGAGGCCCTCCACAACCACTACACCCA GAAGAGCCTCTCCCTGAGCCCTGGAAAG |
| 29 | Amino acid sequence of mutant-I31L+V27L+L4V+Q5K+E1#+E2T+E65D+S66A+E47Q+E70S fusion protein mutant M32 |
| | TEVKVIQPDKSVSVAAGESAILHCTLTSLLPVGPIQWFRGAGPARQLIYNQKEGHFP RVTTVSDATKRSNMDFSISISNITPADAGTYYCVKFRKGSPDTEFKSGAGTELSVRA KPSGGGGSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAV EWESNGQPENNYKTTPPVLDSDGSFFLYSKLT |
| 30 | Nucleic acid sequence of mutant-I31L+V27L+E65D+S66A+E47Q+E70S fusion protein mutant M33 |
| | GAGGAGGAGCTGCAGGTCATCCAGCCTGATAAGTCTGTGAGCGTGGCTGCTGG AGAGTCTGCTATTCTGCATTGTACACTGACATCTCTGCTGCCTGTGGGACCTATT CAGTGGTTTAGAGGAGCTGGACCTGCTAGACAGCTGATCTATAATCAGAAGGAG GGCCATTTTCCTAGAGTGACAACAGTGTCTGACGCCACAAAGAGAAGCAATAT GGATTTTCTATTTCTATTTCTAATATCACCCCTGCTGATGCTGGCACATACTATTG TGTGAAGTTTAGAAAGGGCTCTCCTGATACAGAGTTTAAGTCTGGAGCTGGAAC CGAGCTGTCTGTGAGAGCTAAGCCTTCT GGCGGAGGTGGAAGCGACAAGACCCACACATGTCCCCCCTGTCCCGCTCCTGA ACTGCTGGGAGGCCCTTCCGTGTTCCTGTTCCCCCCTAAGCCCAAGGACACCCT GATGATTTCCAGGACACCCGAGGTGACCTGTGTGGTGGTGGACGTCAGCCACG AGGACCCCGAGGTGAAATTCAACTGGTACGTCGATGGCGTGGAGGTGCACAAC GCTAAGACCAAGCCCAGGGAGGAGCAGTACAATTCCACCTACAGGGTGGTGTC CGTGCTGACCGTCCTCCATCAGGACTGGCTGAACGGCAAAGAGTATAAGTGCA AGGTGAGCAACAAGGCCCTCCCTGCTCCCATCGAGAAGACCATCAGCAAAGCC AAGGGCCAGCCCAGGGAACCTCAAGTCTATACCCTGCCTCCCAGCAGGGAGGA GATGACCAAGAACCAAGTGAGCCTCACATGCCTCGTCAAGGGCTTCTATCCTTC CGATATTGCCGTCGAGTGGGAGTCCAACGGACAGCCCGAGAACAACTACAAGA |
| | CAACACCCCCCGTGCTCGATTCCGATGGCAGCTTCTTCCTGTACTCCAAGCTGA CCGTGGACAAGTCCAGATGGCAACAAGGCAACGTCTTCAGTTGCAGCGTCATG CATGAGGCCCTCCACAACCACTACACCCAGAAGAGCCTCTCCCTGAGCCCTGG AAA |
| 31 | Amino acid sequence of mutant-I31L+V27L+E65D+S66A+E47Q+E70S fusion protein mutant M33 |
| | EEELQVIQPDKSVSVAAGESAILHCTLTSLLPVGPIQWFRGAGPARQLIYNQKEGHF PRVTTVSDATKRSNMDFSISISNITPADAGTYYCVKFRKGSPDTEFKSGAGTELSVR AKPS GGGGSDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSN KALPAPIEKTISKAKGQPREPQVYTLPPSREEMTKNQVSLTCLVKGFYPSDIAVEWE SNGQPENNYKTTPPVLDSDGSFFLYSKLTVDKSRWQQGNVFSCSVMHEALHNHYT QKSLSLSPGK |
| 32 | Amino acid sequence of SIRPα D1 domain of mutant-I31L fusion protein mutant M8 |
| | EEELQVIQPDKSVSVAAGESAILHCTVTSLLPVGPIQWFRGAGPARELIYNQKEGHF PRVTTVSESTKRENMDFSISISNITPADAGTYYCVKFRKGSPDTEFKSGAGTELSVR AKPS |
| 33 | Amino acid sequence of SIRPα D1 domain of mutant-I31L+V27L fusion protein mutant M27 |
| | EEELQVIQPDKSVSVAAGESAILHCTLTSLLPVGPIQWFRGAGPARELIYNQKEGHF PRVTTVSESTKRENMDFSISISNITPADAGTYYCVKFRKGSPDTEFKSGAGTELSVR AKPS |
| 34 | Amino acid sequence of SIRPα D1 domain of mutant-131L+V27L+L4V+Q5K fusion protein mutant M28 |
| | EEEVKVIQPDKSVSVAAGESAILHCTLTSLLPVGPIQWFRGAGPARELIYNQKEGHF PRVTTVSESTKRENMDFSISISNITPADAGTYYCVKFRKGSPDTEFKSGAGTELSVR AKPS |
| 35 | Amino acid sequence of SIRPα D1 domain of mutant-131L+V27L+L4V+Q5K+E1#+E2T fusion protein mutant M29 |
| | TEVKVIQPDKSVSVAAGESAILHCTLTSLLPVGPIQWFRGAGPARELIYNQKEGHFP RVTTVSESTKRENMDFSISISNITPADAGTYYCVKFRKGSPDTEFKSGAGTELSVRA KPS |
| 36 | Amino acid sequence of SIRPα D1 domain of mutant-I31L+V27L+L4V+Q5K+E1#+E2T+E65D+S66A fusion protein mutant M30 |
| | TEVKVIQPDKSVSVAAGESAILHCTLTSLLPVGPIQWFRGAGPARELIYNQKEGHFP RVTTVSDATKRENMDFSISISNITPADAGTYYCVKFRKGSPDTEFKSGAGTELSVRA KPS |
| 37 | Amino acid sequence of SIRPα D1 domain of mutant-I31L+V27L+L4V+Q5K+E1#+E2T+E65D+S66A+E47Q fusion protein mutant M31 |
| | TEVKVIQPDKSVSVAAGESAILHCTLTSLLPVGPIQWFRGAGPARQLIYNQKEGHFP RVTTVSDATKRENMDFSISISNITPADAGTYYCVKFRKGSPDTEFKSGAGTELSVRA KPS |
| 38 | Amino acid sequence of SIRPα D1 domain of mutant-I31L+V27L+L4V+Q5K+E1#+E2T+E65D+S66A+E47Q+E70S fusion protein mutant M32 |
| | mutant M32 TEVKVIQPDKSVSVAAGESAILHCTLTSLLPVGPIQWFRGAGPARQLIYNQKEGHFP RVTTVSDATKRSNMDFSISISNITPADAGTYYCVKFRKGSPDTEFKSGAGTELSVRA KPS |
| 39 | Amino acid sequence of SIRPα D1 of mutant-I31L+V27L+E65D+S66A+E47Q+E70S fusion protein mutant M33 |
| | fusion protein mutant M3 5 EEELQVIQPDKSVSVAAGESAILHCTLTSLLPVGPIQWFRGAGPARQLIYNQKEGHF PRVTTVSDATKRSNMDFSISISNITPADAGTYYCVKFRKGSPDTEFKSGAGTELSVR AKPS |
| 40 | Amino acid sequence of Fc end of human IgG4 |
| | ESKYGPPCPPCPAPEFLGGPSVFLFPPKPKDTLMISRTPEVTCVVVDVSQEDPEVQF |
| | NWYVDGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDIAVEWESNG QPENNYKTTPPVLDSDGSFFLYSRLTVDKSRWQEGNVFSCSVMHEALHNHYTQKS LSLSLGK |
| 41 | Amino acid sequence of SIRP6-IgG4-V6L |
| | EEELQLIQPDKSVSVAAGESAILHCTLTSLLPVGPIQWFRGAGPARQLIYNQKEGHF PRVTTVSDATKRSNMDFSISISNITPADAGTYYCVKFRKGSPDTEFKSGAGTELSVR AKPSGGGGSESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEA LHNHYTQKSLSLSLGK |
| 42 | Amino acid sequence of SIRP6-IgG4-V6L+H56N |
| | EEELQLIQPDKSVSVAAGESAILHCTLTSLLPVGPIQWFRGAGPARQLIYNQKEGNF PRVTTVSDATKRSNMDFSISISNITPADAGTYYCVKFRKGSPDTEFKSGAGTELSVR AKPSGGGGSESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEA LHNHYTQKSLSLSLGK |
| 43 | Amino acid sequence of SIRP6-IgG4-V6L+H56R |
| | EEELQLIQPDKSVSVAAGESAILHCTLTSLLPVGPIQWFRGAGPARQLIYNQKEGRF PRVTTVSDATKRSNMDFSISISNITPADAGTYYCVKFRKGSPDTEFKSGAGTELSVR AKPSGGGGSESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEA LHNHYTQKSLSLSLGK |
| 44 | Amino acid sequence of SIRP6-IgG4-V6L+H56R+A66G |
| | EEELQLIQPDKSVSVAAGESAILHCTLTSLLPVGPIQWFRGAGPARQLIYNQKEGRF PRVTTVSDGTKRSNMDFSISISNITPADAGTYYCVKFRKGSPDTEFKSGAGTELSVR AKPSGGGGSESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEA LHNHYTQKSLSLSLGK |
| 45 | Amino acid sequence of SIRP6-IgG4-V6L+H56R+A66G+K53R |
| | EEELQLIQPDKSVSVAAGESAILHCTLTSLLPVGPIQWFRGAGPARQLIYNQREGRF PRVTTVSDGTKRSNMDFSISISNITPADAGTYYCVKFRKGSPDTEFKSGAGTELSVR AKPSGGGGSESKYGPPCPPCPAPEFLGGPSVFLFPPKPKDTLMISRTPEVTCVVVDV SQEDPEVQFNWYVDGVEVHNAKTKPREEQFNSTYRVVSVLTVLHQDWLNGKEY KCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEA LHNHYTQKSLSLSLGK |
| 46 | Amino acid sequence of CV1-IgG4 |
| | EEELQIIQPDKSVSVAAGESAILHCTITSLFPVGPIQWFRGAGPARVLIYNQRQGPFP RVTTVSETTKRENMDFSISISNITPADAGTYYCIKFRKGSPDTEFKSGAGTELSVRA KPSGGGGSESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCSVMHEAL HNHYTQKSLSLSLGK |
| 47 | Amino acid sequence of SIRPα D1 domain of SIRP6-IgG4-V6L |
| | EEELQLIQPDKSVSVAAGESAILHCTLTSLLPVGPIQWFRGAGPARQLIYNQKEGHF PRVTTVSDATKRSNMDFSISISNITPADAGTYYCVKFRKGSPDTEFKSGAGTELSVR AKPS |
| 48 | Amino acid sequence of SIRPα D1 domain of SIRP6-IgG4-V6L+H56N |
| | EEELQLIQPDKSVSVAAGESAILHCTLTSLLPVGPIQWFRGAGPARQLIYNQKEGNF |
| | PRVTTVSDATKRSNMDFSISISNITPADAGTYYCVKFRKGSPDTEFKSGAGTELSVR AKPS |
| 49 | Amino acid sequence of SIRPα D1 domain of SIRP6-IgG4-V6L+H56R |
| | EEELQLIQPDKSVSVAAGESAILHCTLTSLLPVGPIQWFRGAGPARQLIYNQKEGRF PRVTTVSDATKRSNMDFSISISNITPADAGTYYCVKFRKGSPDTEFKSGAGTELSVR AKPS |
| 50 | Amino acid sequence of SIRPα D1 domain of SIRP6-IgG4-V6L+H56R+A66G: |
| | EEELQLIQPDKSVSVAAGESAILHCTLTSLLPVGPIQWFRGAGPARQLIYNQKEGRF PRVTTVSDGTKRSNMDFSISISNITPADAGTYYCVKFRKGSPDTEFKSGAGTELSVR AKPS |
| 51 | Amino acid sequence of SIRPα D1 domain of SIRP6-IgG4-V6L+H56R+A66G+K53R |
| | EEELQLIQPDKSVSVAAGESAILHCTLTSLLPVGPIQWFRGAGPARQLIYNQREGRF PRVTTVSDGTKRSNMDFSISISNITPADAGTYYCVKFRKGSPDTEFKSGAGTELSVR AKPS |

The following examples are intended to further illustrate the present invention and should not be construed as limiting the present invention. The examples do not include a detailed description of conventional methods or conventional methods in the art, such as methods of preparing nucleic acid molecules, methods of constructing vectors and plasmids, methods of inserting genes encoding proteins into such vectors and plasmids or introducing plasmids into host cells, and methods of culturing host cells. Such methods are well known to those of ordinary skill in the art and have been described in numerous publications, including Sambrook, J., Fritsch, E.F. and Maniais, T. (1989) Molecular Cloning: A Laboratory Manual, 2nd edition, Cold Spring Harbor Laboratory Press. Experimental procedures without specified conditions in the following examples, are generally carried out under conventional conditions, or under conditions recommended by the manufacturer. Unless otherwise stated, percentages and parts are by weight.

The experimental materials used in the following examples and their sources as well as the methods of formulating the experimental reagents are specifically described below. Unless otherwise stated, they are all commercially available.

### Materials:

HEK293F cells: purchased from Thermo Fisher Scientific.
pcDNA3.4: purchased from Thermo Fisher Scientific.
Daudi cell line: purchased from ATCC, catalog No. CCL-213^{™}.

### Reagents:

Sodium carbonate buffer: 1.59 g of Na₂CO₃ and 2.93 g of NaHCO₃ were dissolved in 1 L of pure water.
Phosphate buffered saline: abbreviated as PBST, the formula is as follows: 0.2 g of KH₂PO₄, 2.9 g of Na₂HPO₄·12H₂O, 8.0 g of NaCl, 0.2 g of KCl, and 0.5 mL of Tween-20; brought to 1 L by adding pure water.

Color developing solution: substrate color developing solution A: 13.6 g of sodium acetate trihydrate, 1.6 g of citric acid monohydrate, 0.3 mL of 30% hydrogen peroxide and 500 mL of pure water; substrate color developing solution B: 0.2 g of disodium ethylenediaminetetraacetic acid, 0.95 g of citric acid monohydrate, 50 mL of glycerol, 0.15 g of TMB dissolved in 3 mL of DMSO, and 500 mL of pure water; equal volumes of solution A and solution B were mixed before use.
Stop solution: 2 M sulfuric acid solution.
HRP-labeled goat anti-mouse secondary antibody: purchased from Sigma, catalog No. SAB3701283.
RPMI-1640 medium: purchased from Thermo Fisher Scientific.
Free Style 293 Expression Medium: purchased from Thermo Fisher Scientific. Fetal bovine serum: purchased from Thermo Fisher Scientific.
CytoTox 96 Non-Radioactive Cytotoxicity Assay: purchased from Promega, catalog No. G1780.
Propidium Iodide: purchased from Sigma, catalog No. 81845-100MG.
Annexin V apoptosis detection kit: purchased from BD Biosciences, catalog No. 556570.

### Experimental instruments:

Microplate reader: purchased from Molecular Devices, model SpectraMax 190.
Multifunctional microplate reader: purchased from Molecular Devices, model SpectraMax M5.
CO₂ shaking incubator: purchased from INFORS.
Unless otherwise stated, the DNA sequences used in the following examples were all synthesized by Shanghai Sangon Biotech Co., Ltd.

### Example 1. Affinity Comparison of Wild-Type Human SIRPα-Fc Fusion Protein and Mouse SIRPα-Fc Fusion Protein

**1.1 Preparation of wild-type human SIRPα-Fc fusion protein SIRP-Fc-IgG1** Human SIRPα binds to its ligand CD47 mainly via the first domain, Domain 1, at the N-terminus (SIRPα D1 Domain for short hereinafter), and the D1 Domain is polymorphic (reference: Barclay A N. Signal regulatory protein alpha (SIRPα)/CD47 interaction and function[J]. Current Opinion in Immunology, 2009, 21(1): 47-52). In this example, the D1 domain of human SIRPα (NCBI accession No. CAA71403) was used, and its amino acid sequence is set forth in SEQ ID NO: 1. The amino acid sequence of the Fc segment of the heavy chain of human IgG1 is set forth in SEQ ID NO: 2.

The DNA of SEQ ID NO: 1 was linked to the human IgG1 heavy chain Fc fragment gene by recombinant PCR via a GGGGS linker. Then the fusion gene was constructed into the pcDNA3.4 expression vector, and the expression vector was transferred into HEK293F cells by PEI transfection to express the fusion protein. The HEK293F cells were cultured in Free Style 293 Expression Medium. The transfected HEK293F cells were incubated in a CO₂ shaking incubator for 5 days and centrifuged, and the cell supernatant was collected. The fusion protein in the supernatant was purified by protein A affinity chromatography, and the resulting protein was designated SIRP-Fc-IgG1. The nucleic acid sequence and amino acid sequence of the protein are set forth in SEQ ID NO: 4 and SEQ ID NO: 5, respectively.

### 1.2 Preparation of wild-type mouse SIRPα-Fc fusion protein mSIRP-Fc-IgG1

It was reported that NOD wild-type mouse SIRPα has abnormally high affinity for human CD47 (reference: Kwong L S, Brown M H, Barclay A N, et al. Signal-regulatory protein α from the NOD mouse binds human CD47 with an exceptionally high affinity--implications for engraftment of human cells[J]. Immunology, 2014, 143(1): 61-67). The amino acid sequence of the NOD wild-type mouse SIRPα D1 domain is set forth in SEQ ID NO: 6.

The DNA of SEQ ID NO: 6 was linked to the human IgG1 heavy chain Fc fragment gene by recombinant PCR via a GGGGS linker, and then the fusion gene was constructed into the pcDNA3.4 expression vector. The protein was expressed and purified using the method described in Example 1.1, and the resulting fusion protein was designated mSIRP-Fc-IgG1. The nucleic acid sequence and amino acid sequence of the protein are set forth in SEQ ID NO: 7 and SEQ ID NO: 8, respectively.

### 1.3 Preparation of SIRPα-Fc fusion protein's ligand CD47-His

The sequence information of the N-terminal IgV-like domain of the CD47 extracellular segment is from https://www.uniprot.org/uniprot/Q08722, and its amino acid sequence is set forth in SEQ ID NO: 9.

The DNA of SEQ ID NO: 9 was linked by recombinant PCR to a linker and a coding sequence encoded by a polyhistidine tag, and then the fusion gene was constructed into the pcDNA3.4 expression vector. The recombinant protein was expressed using the method described above. The recombinant protein in the supernatant was purified by nickel affinity chromatography, and the resulting protein was designated CD47-His. The nucleic acid sequence and amino acid sequence of the protein are set forth in SEQ ID NO: 10 and SEQ ID NO: 11, respectively.

### 1.4 Preparation of positive control antibodies

### 1.4.1 Preparation of positive control antibody magrolimab

The amino acid sequences of the heavy and light chain variable regions of the positive control antibody magrolimab (anti-CD47 monoclonal antibody) are from "WHO Drug Information, Vol. 32, No. 4, 2018". The synthesized heavy chain variable region gene of magrolimab was linked to the human IgG1 heavy chain constant region gene to obtain a full-length heavy chain gene, which was designated magrolimab-HC-IgG1. The light chain variable region gene of magrolimab was linked to the human Kappa chain constant region gene to obtain a full-length light chain gene, which was designated magrolimab-LC. The magrolimab-HC-IgG1 gene and the magrolimab-LC gene were each constructed into the pcDNA3.4 expression vector, and the antibody was expressed and purified using the method described in Example 1.1, and the resulting antibody was designated magrolimab-IgG1. The amino acid sequences of the heavy chain and light chain of the antibody are set forth in SEQ ID NO: 12 and SEQ ID NO: 13, respectively.

### 1.4.2 Preparation of positive control antibody anti-CD47B-Hu

Anti-CD47B-Hu is another anti-human CD47 monoclonal antibody, and its amino acid sequences of the heavy chain variable region and the light chain variable region are SEQ ID NO: 61 and SEQ ID NO: 62 in Chinese Patent Application 202010357134.4, respectively. The synthesized heavy chain variable region gene of anti-CD47B-Hu was linked to the human IgG1 heavy chain constant region gene to obtain a full-length heavy chain gene, which was designated anti-CD47B-Hu-HC-IgG1; the light chain variable region gene of anti-CD47B-Hu was linked to the human Kappa chain constant region gene to obtain a full-length light chain gene, which was designated anti-CD47B-Hu-LC. The anti-CD47B-Hu-HC-IgG1 gene and the anti-CD47B-Hu-LC gene were each constructed into the pcDNA3.4 expression vector, and the antibody was expressed and purified using the method described in Example 1.1, and the resulting antibody was designated anti-CD47B-Hu-IgG1. The amino acid sequences of the heavy chain and light chain of the antibody are set forth in SEQ ID NO: 14 and SEQ ID NO: 15, respectively.

### 1.5 ELISA assay for relative affinity of wild-type human SIRPα-Fc fusion protein and mouse SIRP-Fc fusion protein

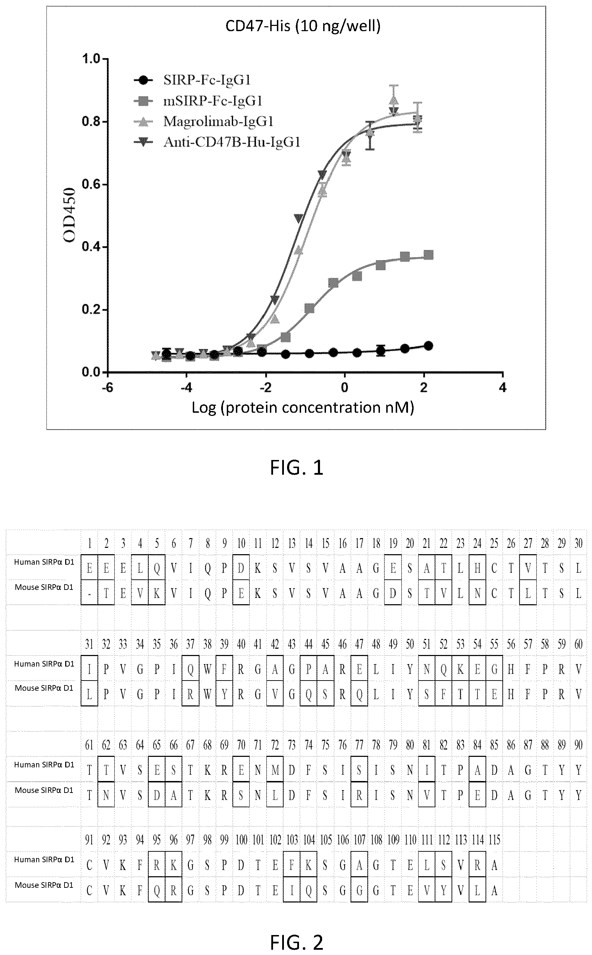

The relative affinity of SIRP-Fc-IgG1 prepared in Example 1.1, mSIRP- Fc- IgG 1 prepared in Example 1.2 and magrolimab-IgG1 and anti-CD47B-Hu-IgG1 prepared in Example 1.4 for CD47 was determined by ELISA. Method: Human CD47-His prepared in Example 1.3 was diluted to 100 ng/mL with a sodium carbonate buffer, and then the dilution was added to a microplate at 100 µL per well. The plate was incubated at room temperature for 2 h and washed with PBST. The plate was blocked by adding PBST containing 1% bovine serum albumin (BSA) to each well and incubated at room temperature for 1 h. The plate was washed twice with PBST, and serially diluted antibodies or fusion proteins were added. The plate was incubated for half an hour and washed twice with PBST. A properly diluted HRP-labeled goat anti-human (Fc-Specific) secondary antibody was added, and the plate was incubated for half an hour. After the plate was washed, a color developing solution was added for color development, and the color development was terminated with a stop solution. The OD450 was read using on a microplate reader. The data were analyzed in GraphPad Prism 6 and plotted, and EC₅₀ was calculated. The results are shown in FIG. 1.

FIG. 1 shows that mSIRP-Fc-IgG1, magrolimab-IgG1 and anti-CD47B-Hu-IgG1 were all able to bind to CD47 effectively, the EC₅₀ values were 0.1565 nM, 0.1119 nM and 0.05584 nM, respectively, and the heights of the top plateaus were 0.3682, 0.8359 and 0.7946, respectively. The results indicate that the relative affinities of magrolimab-IgG1 and anti-CD47B-Hu-IgG1 for CD47 were comparable and that the relative affinities of both for CD47 were significantly higher than that of mSIRP-Fc-IgG1. Under these conditions, SIRP-Fc-IgG1 was unable to bind to CD47 effectively.

### Example 2. Preparation of SIRPα-Fc Fusion Protein Mutants and Relative Affinity Screening

### 2.1 Preparation of SIRPα-Fc fusion protein mutants

BLAST (Basic Local Alignment Search Tool) analysis showed that there were some differences between the human SIRPα D1 domain and the NOD mouse SIRPα D1 domain in terms of amino acid sequence. Their sequences differed by a single amino acid at some positions and by several consecutive amino acids at some positions (the sequence alignment is shown in FIG. 2). On the basis of the SIRP- Fc- IgG 1 gene, the amino acid sequences at the positions where the differences were present between the human and mouse SIRPα D1 domains were mutated into the amino acid sequences of the corresponding positions of the mouse domain, and thus a series of SIRP- Fc- IgG 1 mutants shown in Table 2 were constructed. These SIRP- Fc- IgG 1 mutants were expressed and purified using the method described in Example 1.1. These mutations were designated as follows: if the E at position 1 was deleted and the E at position 1 was mutated into T, the mutant was designated Mutant-E1#+E1T; if the L at position 4 was mutated into V and the Q at position 5 was mutated into K, the mutant was designated mutant-L4V+Q5K; the other mutants were also designated in the way.

### 2.2 ELISA assay for relative affinity of SIRPα-Fc fusion protein mutants

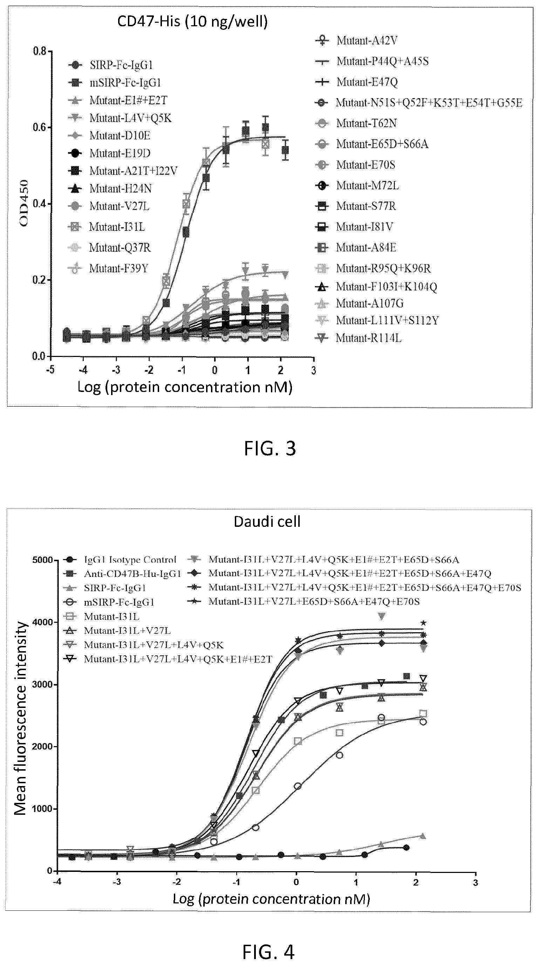

The relative affinity of the prepared SIRP-Fc-IgG1 mutants for CD47 was determined by ELISA as described in Example 1.5, and the results are shown in FIG. 3 and Table 2.

**Table 2. ELISA assay for relative affinity**

| Protein No. | Protein | EC₅₀ (nM) | Height of top plateau |
|---|---|---|---|
| / | SIRP-Fc-IgG1 | 3.417 | 0.08551 |
| / | mSIRP-Fc-IgG1 | 0.1256 | 0.5785 |
| **M1** | **Mutant-E1#+E2T** | **0.4188** | **0.165** |
| **M2** | **Mutant-L4V+Q5K** | **0.1982** | **0.2255** |
| M3 | Mutant-D10E | 0.1211 | 0.06791 |
| M4 | Mutant-E19D | 0.1585 | 0.0836 |
| **M5** | **Mutant-A21T+I22V** | **0.2798** | **0.1171** |
| **M6** | **Mutant-H24N** | **1.564** | **0.09228** |
| **M7** | **Mutant-V27L** | **0.1253** | **0.1492** |
| **M8** | **Mutant-I31L** | **0.07086** | **0.5704** |
| M9 | Mutant-Q37R | NA | 0.05772 |
| M10 | Mutant-F39Y | NA | 0.1474 |
| M11 | Mutant-A42V | 0.2064 | 0.06995 |
| M12 | Mutant-P44Q+A45S | 0.1869 | 0.07832 |
| **M13** | **Mutant-E47Q** | **0.2408** | **0.09813** |
| M14 | Mutant-N51S+Q52F+K53T+E54T+G55E | 0.4564 | 0.05401 |
| M15 | Mutant-T62N | 2.784 | 0.07574 |
| **M16** | **Mutant-E65D+S66A** | **0.1222** | **0.152** |
| **M17** | **Mutant-E70S** | **0.1341** | **0.1148** |
| M18 | Mutant-M72L | NA | 0.05854 |
| M19 | Mutant-S77R | NA | 0.08005 |
| **M20** | **Mutant-I81V** | **0.1947** | **0.1138** |
| **M21** | **Mutant-A84E** | **0.7016** | **0.09018** |
| M22 | Mutant-R95Q+K96R | NA | 0.05534 |
| **M23** | **Mutant-F103I+K104Q** | **1.886** | **0.08813** |
| M24 | Mutant-A107G | 0.1506 | 0.07745 |
| M25 | Mutant-L111V+S112Y | 3.769 | 0.08204 |
| **M26** | **Mutant-R114L** | **1.732** | **0.09071** |

| | | | |
|---|---|---|---|
| Note: "#" indicates deleted or absent; E2T indicates the E at position 2 being mutated into T, and so on; "+" indicates simultaneous mutation or coexistence. | | | |

In this example, the effect of each point mutation or each set of point mutations on the binding affinity of SIRP for CD47 was analyzed in detail. FIG. 3 and Table 2 show that the mutants M1, M2, M5, M6, M7, M8, M13, M16, M17, M20, M21, M23 and M26 had significantly higher relative affinity than SIRP-Fc-IgG1 (which was determined based on the fact that they had smaller EC₅₀ and higher top plateaus than SIRP-Fc-IgG1; EC₅₀ is in inverse proportion to relative affinity, and height of top plateau is in direct proportion to relative affinity).

### Example 3. Screening for Mutation Combinations That Increase SIRPα Affinity

### 3.1 Preparation of SIRPα-Fc fusion protein mutants comprising mutation combinations

Among the mutants obtained by screening in Example 2, mutant-I31L has the highest relative affinity. Other mutation modes of mutants obtained by screening in Example 2 were further introduced into mutant-I31L to construct mutants M27-M33 comprising mutation combinations shown in Table 3. These mutants were expressed and purified using the method described in Example 1.1.

### 3.2 Flow cytometry assay for binding capacity of SIRPα-Fc fusion protein mutants comprising mutation combinations to Daudi cells

The binding capacity of the prepared SIRP-Fc-IgG1 mutants to CD47 on the surfaces of Daudi cells (human lymphoma cells) was determined by flow cytometry. Experimental procedure: After being counted, Daudi cells were inoculated into a 96-well round-bottom culture plate at 2E5 cells/150 µL by using a PBS solution containing 1% bovine serum albumin (BSA). To the 96-well plate described above were added 50 µL of SIRPα-Fc fusion protein mutants serially diluted with the PBS solution. The plate was incubated at room temperature for 1 h and then centrifuged, and the supernatant was discarded. Then the cells were washed twice with PBS. An FITC-labeled goat anti-human (Fc-Specific) antibody (1:1000 diluted with PBS containing 1% BSA) was added. The plate was incubated at room temperature for half an hour and centrifuged, and the cells were washed and then tested for mean fluorescence intensity (MFI) in the FITC channel on a flow cytometer (CytoFLEX Cytometer System, purchased from Beckman Coulter). The experimental data were processed using the software provided with the flow cytometer, and the mean fluorescence intensity was calculated. The data were analyzed and plotted in GraphPad Prism 6, and EC₅₀ was calculated. The experimental results are shown in FIG. 4 and Table 3 (IgG1 Isotype Control in FIG. 4 refers to an isotype antibody which does not bind to CD47).

**Table 3. Flow cytometry assay for cell binding capacity**

| Protein No. | Protein | EC₅₀ (nM) | Height of top plateau |
|---|---|---|---|
| / | Anti-CD47B-Hu-IgG1 | 0.1894 | 3069 |
| / | SIRP-Fc-IgG1 | 29.05 | 698.7 |
| / | mSIRP-Fc-IgG1 | 1.201 | 2578 |
| M8 | Mutant-I31L | 0.2281 | 2463 |
| M27 | Mutant-I31L+V27L | 0.2077 | 2858 |
| M28 | Mutant-131L+V27L+L4V+Q5K | 0.2113 | 2876 |
| M29 | Mutant-I31L+V27L+L4V+QSK+E1#+E2T | 0.2122 | 2888 |
| M30 | Mutant-131L+V27L+L4V+Q5K+E1#+E2T+E 65D+S66A | 0.1536 | 3781 |
| M31 | Mutant-131L+V27L+L4V+Q5K+E1#+E2T+E 65D+S66A+E47Q | 0.1347 | 3686 |
| M32 | Mutant-131L+V27L+L4V+Q5K+E1#+E2T+E 65D+S66A+E47Q+E70S | 0.1386 | 3849 |
| M33 | Mutant-I31L+V27L+ E65D+S66A+E47Q+E70S | 0.1317 | 3913 |

FIG. 4 and Table 3 show that the addition of some site mutations (such as I31L, V27L, E65D+S66A, E47Q, and E70S) could heighten the top plateaus of the SIRP- Fc- IgG 1 mutants or decrease their EC₅₀ values, suggesting that mutants with additional mutations had increased capacity to bind to cells. The effect of adding the two sets of mutations L4V+Q5K and E1#+E2T on the affinity of the mutants was not significant. The affinity of mutant M33 without these two sets of mutations for Daudi cells was substantially equivalent to that of M32. The mutation combinations M27-M33 all had smaller EC₅₀ (nM) than M8 and higher top plateaus than M8, showing increased capacity to bind to Daudi cells compared to M8. The mutation combinations M30-M33 all had smaller EC₅₀ than anti-CD47B-Hu-IgG1 and significantly higher top plateaus than anti-CD47B-Hu-IgG1, indicating that the four mutants had significantly greater capacity to bind to Daudi cells than anti-CD47B-Hu-IgG1.

### Example 4. Preparation and Screening of Mutants Having Further Increased SIRP Mutant Affinity

### 4.1 Preparation of mutants having further increased SIRP mutant affinity

Here, the wild-type SIRP in SIRP-Fc-IgG1 was linked to the Fc end of human IgG4 via a linker (GGGGS), and the resulting recombinant protein was designated SIRP-IgG4. The M33 mutant (containing 6 mutation sites and therefore hereinafter referred to as SIRP6) was linked to the Fc end of human IgG4 via a linker (GGGGS), and the resulting recombinant protein was designated SIRP6-IgG4. Analysis of the disclosed crystal structures (reference: D Hatherley, Graham S C, Turner J, et al. Paired receptor specificity explained by structures of signal regulatory proteins alone and complexed with CD47.[J]. Mol. Cell, 2008, 31(2):266-277) reveals that amino acid residue such as V6, Q52, K53, H56 and A66 are at or near the interface between SIRP and CD47; they may play a critical role in the binding of SIRP to CD47. Here, in this example, a series of mutants (V6L, Q52E, Q52N, Q52R, K53R, H56N, H56Q, H56R, A66G, A66L and A66T) were prepared by site-directed mutagenesis of these sites on the basis of SIRP6-IgG4. Here, theses mutations were done based on the fact that each amino acid is mutated into an amino acid of similar nature or of similar size. These mutants were expressed and purified using the method described in Example 1.1. The amino acid sequence of the Fc end of human IgG4 is set forth in SEQ ID NO: 40.

### 4.2 ELISA assay for relative affinity of SIRP6 mutants

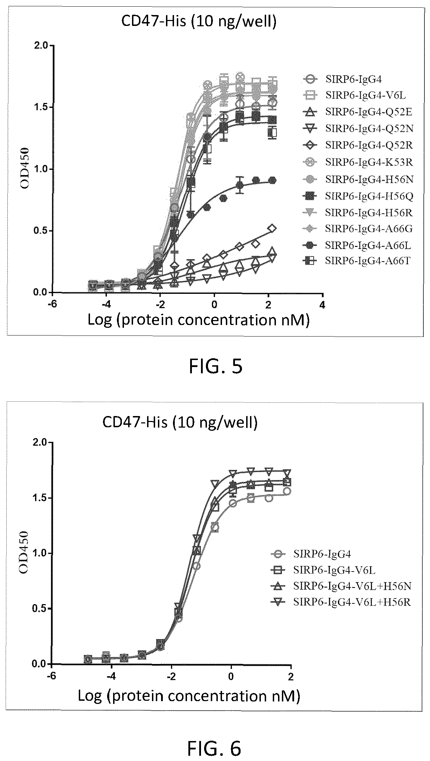

The relative affinity of the mutants of the SIRP6 series prepared above for CD47 was determined by ELISA as described in Example 1.5, and the results are shown in FIG. 5 and Table 4.

**Table 4. ELISA assay for relative affinity**

| Mutant | EC₅₀ (nM) | Top |
|---|---|---|
| SIRP6-IgG4 | 0.04785 | 1.526 |
| SIRP6-IgG4-V6L | 0.03472 | 1.705 |
| SIRP6-IgG4-Q52E | NA | NA |
| SIRP6-IgG4-Q52N | NA | NA |
| SIRP6-IgG4-Q52R | NA | NA |
| SIRP6-IgG4-K53R | 0.0384 | 1.697 |
| SIRP6-IgG4-H56N | 0.04311 | 1.634 |
| SIRP6-IgG4-H56Q | 0.0641 | 1.438 |
| SIRP6-IgG4-H56R | 0.05096 | 1.603 |
| SIRP6-IgG4-A66G | 0.04341 | 1.625 |
| SIRP6-IgG4-A66L | 0.05077 | 0.9131 |
| SIRP6-IgG4-A66T | 0.07483 | 1.385 |

The ELISA results (FIG. 5 and Table 4) show that the SIRP6-IgG4 mutant with V6L, K53R, H56N, H56R or A66G had a higher Top and/or smaller EC₅₀ than SIRP6-IgG4, indicating that these mutations were able to increase the binding capacity of SIRP6-IgG4 to CD47.

### Example 5. Screening for Mutation Combinations That Further Increase Affinity of SIRP6 Mutants

### 5.1 Preparation of SIRP6 mutants comprising mutation combinations

Among the mutants obtained by screening in Example 4, SIRP6-IgG4-V6L had the highest Top. The H56N or H56R mutation was further introduced into SIRP6-IgG4-V6L (the amino acid sequence is set forth in SEQ ID NO: 41), and the resulting mutant was designated SIRP6-IgG4-V6L+H56N (the amino acid sequence is set forth in SEQ ID NO: 42) or SIRP6-IgG4-V6L+H56R (the amino acid sequence is set forth in SEQ ID NO: 43). These mutants were expressed and purified using the method described in Example 1.1.

### 5.2 ELISA assay for relative affinity of SIRP6 mutants comprising mutation combinations

The relative affinity of SIRP mutants comprising mutation combinations described above was determined by ELISA as described in Example 1.5.

**Table 5. ELISA assay for relative affinity**

| Mutant | EC₅₀ (nM) | Top |
|---|---|---|
| SIRP6-IgG4 | 0.05453 | 1.541 |
| SIRP6-IgG4-V6L | 0.04289 | 1.634 |
| SIRP6-IgG4-V6L+H56N | 0.04531 | 1.668 |
| SIRP6-IgG4-V6L+H56R | 0.03933 | 1.756 |

The ELISA results (FIG. 6 and Table 5) show that the SIRP6-IgG4 mutant with the combination V6L+H56R had a higher Top and smaller EC₅₀ than that with the combination V6L+H56N, indicating that H56R was more effective than H56N in increasing the binding capacity of SIRP6-IgG4 mutants to CD47.

The A66G mutation was further introduced into SIRP6-IgG4-V6L+H56R, and the resulting mutant was designated SIRP6-IgG4-V6L+H56R+A66G (the amino acid sequence is set forth in SEQ ID NO: 44). These mutants were expressed and purified using the method described in Example 1.1. The relative affinity of SIRP mutants was determined by ELISA as described in Example 1.5.

**Table 6. ELISA assay for relative affinity**

| Mutant | EC₅₀ (nM) | Top |
|---|---|---|
| SIRP6-IgG4 | 0.05453 | 1.541 |
| SIRP6-IgG4-V6L | 0.04289 | 1.634 |
| SIRP6-IgG4-V6L+H56R | 0.04526 | 1.703 |
| SIRP6-IgG4-V6L+H56R+A66G | 0.03391 | 1.771 |

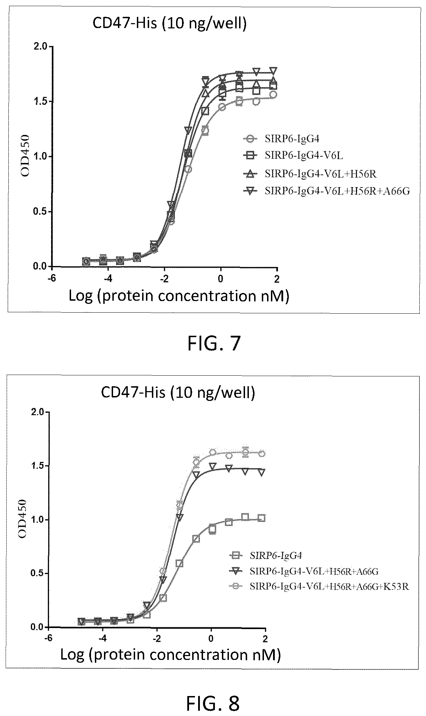

The ELISA results (FIG. 7 and Table 6) show that the SIRP6-IgG4 mutant with the combination V6L+H56R+A66G had a higher Top and smaller EC₅₀ than that with the combination V6L+H56R, indicating that the A66G mutation further increased the binding capacity of the SIRP6-IgG4-V6L+H56R mutant to CD47.

The K53R mutation was further introduced into SIRP6-IgG4-V6L+H56R+A66G, and the resulting mutant was designated SIRP6-IgG4-V6L+H56R+A66G+K53R (the amino acid sequence is set forth in SEQ ID NO: 45). These mutants were expressed and purified using the method described in Example 1.1. The relative affinity of SIRP mutants was determined by ELISA as described in Example 1.5.

**Table 7. ELISA assay for relative affinity**

| Mutant | EC₅₀ (nM) | Top |
|---|---|---|
| SIRP6-IgG4 | 0.05549 | 1.014 |
| SIRP6-IgG4-V6L+H56R+A66G | 0.03633 | 1.483 |
| SIRP6-IgG4-V6L+H56R+A66G+K53R | 0.03441 | 1.635 |

The ELISA results (FIG. 8 and Table 7) show that the SIRP6-IgG4 mutant with the combination V6L+H56R+A66G+K53R had a higher Top and smaller EC₅₀ than that with the combination V6L+H56R+A66G, indicating that the K53R mutation further increased the binding capacity of the SIRP6-IgG4-V6L+H56R mutant to CD47. Here, SIRP6-IgG4-V6L+H56R+A66G+K53R was denoted by SIRP10-IgG4.

### Example 6. Comparison of Affinity of Mutants of SIRP series

A high-affinity mutant CV1 of SIRP was found in the published document (Engineered SIRPα Variants as Immunotherapeutic Adjuvants to Anticancer Antibodies[J]. Science, 2013, 341(6141):88-91). It is linked to the Fc-end of human IgG4 via a linker (GGGGS), and the resulting recombinant protein was designated CV1-IgG4 (the amino acid sequence is set forth in SEQ ID NO: 46). These mutants were expressed and purified using the method described in Example 1.1.

Biacore is a method commonly used for molecular interaction analysis based on the principle of surface plasmon resonance (SPR for short). Here, Biacore was used to determine the affinity of the SIRP mutants described above and anti-CD47 antibodies for the extracellular end of CD47. Experimental method and procedure: The SIRP mutant or anti-CD47 antibodies were diluted to a concentration of 1 µg/mL with HBS-EP⁺ buffer (pH 7.4, purchased from Cytiva, catalog No. BR-1006-69). CD47-His was diluted to 25 nM, 12.5 nM, 6.25 nM, 3.125 nM, 1.5625 nM and 0.78125 nM, and a zero concentration was set. A 6 M solution of guanidine hydrochloride was used as a regeneration buffer. Sensor Chip Protein A (purchased from Cytiva, catalog No. 29-1275-55) was used to capture antibody on Biacore 8K (purchased from GE Healthcare). Then CD47-His was used as an analyte and allowed to flow over the chip, and an association-dissociation curve was obtained. After the chip was regenerated with the regeneration buffer, another cycle was carried out. The data were analyzed using Biacore 8K Evaluation Software.

**Table 8. Determination of equilibrium dissociation constants (affinity) for SIRP mutants and anti-CD47 antibodies**

| **Mutant/antibody** | **ka (1/Ms)** | **kd (1/s)** | **KD (M)** |
|---|---|---|---|
| SIRP-IgG4 | 2.79E+06 | 6.19E-03 | 2.22E-09 |
| SIRP6-IgG4 | 1.68E+06 | 3.77E-04 | 2.25E-10 |
| CV1-IgG4 | 1.88E+06 | 1.14E-04 | 6.05E-11 |
| SIRP10-IgG4 | 1.75E+06 | 7.90E-05 | 4.51E-11 |
| Magrolimab-IgG1 | 1.06E+06 | 1.37E-04 | 1.30E-10 |
| Anti-CD47B-Hu-IgG1 | 1.16E+06 | 1.20E-04 | 1.04E-10 |

The Biacore results (Table 8) show that the affinity of SIRP6-IgG4 was an order of magnitude higher than that of the wild-type SIRP-IgG4, and was substantially comparable to that of anti-CD47 monoclonal antibodies (magrolimab-IgG1 and anti-CD47B-Hu-IgG1). Among all the mutants, the SIRP10-IgG4 had the highest affinity, higher than CV1-IgG4, which is mainly reflected in the slowest dissociation rate (the smallest kd) of SIRP10-IgG4.

### Example 7. Determination of ADCC of Mutations Described Above

The SIRP mutant can bind to CD47 on the surface of a cell, and the Fc segment binds to the Fc receptor on the surface of an effector cell (NK cell and the like), and thus the direct killing of target cells by effector cells can be mediated, which is known as antibody-dependent cell-mediated cytotoxicity (ADCC). In this example, the ADCC activity of the mutants described above was determined.

The method is specifically as follows: 2% fetal bovine serum was added to RPMI-1640 (Gibco, catalog No. 11835-030). Target Daudi cells and human peripheral blood mononuclear cells (PBMCs) used as effector cells were mixed in a ratio of 1:25 in the medium. The cell suspension was inoculated into a 96-well round-bottom plate at 150 µL/well such that 2 × 10⁴ Daudi cells and 5 × 10⁵ PBMCs were contained in each well. 50 µL of serially diluted antibody mutants was added. The plate was incubated in a cell incubator overnight. 50 µL of the supernatant of the cell culture was transferred to a new 96-well plate, and to each well was added 50 µL of the CytoTox 96 Non-Radioactive Cytotoxicity Assay (Promega, catalog No. G1780) reaction solution. After half an hour, a stop solution was added to terminate the reaction. The OD490 values of the 96-well plate were read on SpectraMax 190. ADCC was calculated using the formula recommended by Promega: cytotoxicity (%) = (experimental - effector spontaneous - target spontaneous)/(target maximum - target spontaneous) × 100. Then the data were analyzed and plotted in GraphPad Prism 7, and EC₅₀ was calculated.

**Table 9**

| Antibody | EC₅₀ (nM) | Top |
|---|---|---|
| SIRP-Fc-IgG1 | 2.059 | 36.85 |
| SIRP6- IgG1 | 0.0781 | 52.51 |
| SIRP6-IgG4 | 583.3 | NA |
| Anti-CD47B-Hu-IgG1 | 0.1974 | 29.01 |

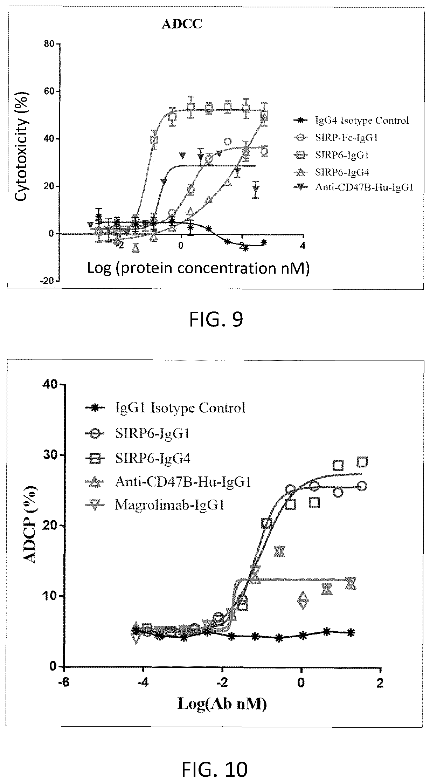

The Top here means the highest degree of ADCC obtained by curve fitting. As shown in FIG. 9 and Table 9, the SIRP6-IgG1 mutant had the smallest EC₅₀ (0.0781 nM) and the highest Top (52.51) among the fusion proteins and antibodies described above, and thus its ADCC was the strongest. IgG4 Isotype control is a control antibody which does not bind to a specific target.

### Example 8. Determination of ADCP of Mutations Described Above

The SIRP mutant can bind to CD47 on the surface of a cell, and the Fc segment binds to the Fc receptor on the surface of an effector cell (macrophage and the like), and thus the phagocytosis of target cells by effector cells can be mediated, which is known as antibody-dependent cellular phagocytosis (ADCP). In this example, the ADCP activity of the mutants described above was determined.

Monocytes were isolated from human PBMCs by using the adherence method and were induced to differentiate into macrophages in a RPMI-1640 medium (containing 10% fetal bovine serum and 50 ng/mL recombinant human granulocyte-macrophage colony stimulating factor). Daudi cells or Jurkat cells (purchased from ATCC) were collected by centrifugation, washed twice with PBS, and then stained with CFSE (5(6)-carboxyfluorescein diacetate N-succinimidyl ester). The macrophages produced by induction were detached by being treated with trypsin-EDTA. The two types of cells described above were then mixed well in a ratio, and the mixture was inoculated into a 96-well round-bottom plate such that 0.3 million macrophages and 0.1 million Daudi cells were contained in each well. Serially diluted fusion proteins or monoclonal antibodies were added to the 96-well plate, and the plate was incubated in a cell incubator for 3 h. The cells in the 96-well plate were washed with PBS, and then the macrophages were stained with APC Mouse Anti-Human CD11b (BD Biosciences, catalog No. 550019) for half an hour. The cells were fixed with paraformaldehyde, and the fluorescence distribution and intensity of the cells in each well were determined on CytoFLEX Cytometer System (Beckman Coulter). The data were processed, analyzed and plotted in Graphpad Prism 7, and EC₅₀ was calculated.

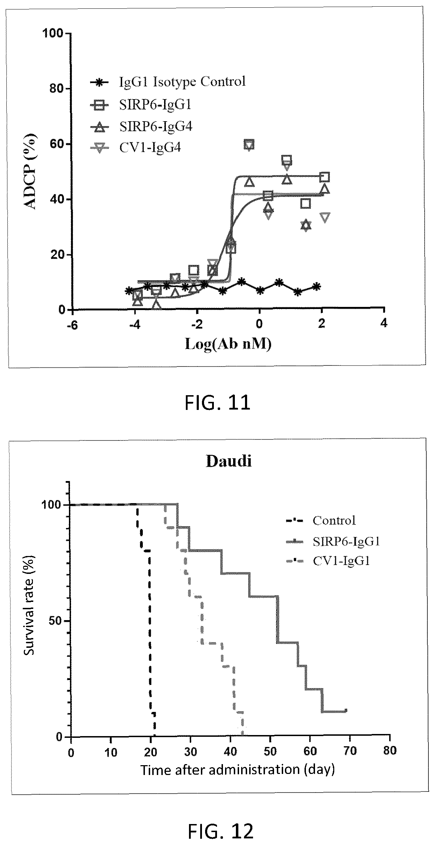

The Top here means the highest degree of ADCP obtained by curve fitting. Daudi cells were used as the target cells in FIG. 10, and Jurkat cells in FIG. 11. As shown in FIG. 10, the EC₅₀ values of the ADCP of SIRP6-IgG1, SIRP6-IgG4, anti-CD47B-Hu-IgG1 and Magrolimab-IgG1 were 0.067 nM, 0.092 nM, 0.019 nM and 0.018 nM, respectively, and their Tops were 25.61, 27.56, 12.46 and 12.49, respectively. Although SIRP6-IgG1 and SIRP6-IgG4 had smaller EC₅₀ values than CD47B-Hu-IgG1 and Magrolimab-IgG1, SIRP6-IgG1 and SIRP6-IgG4 had higher Tops. This indicates that SIRP6-IgG1 and SIRP6-IgG4 had significantly higher ADCP than anti-CD47 monoclonal antibodies when at high concentrations. IgG4 Isotype control is a control antibody which does not bind to a specific target.

As shown in FIG. 11, the EC₅₀ values of the ADCP of SIRP6-IgG1, SIRP6-IgG4 and CV1-IgG4 were 0.14 nM, 0.077 nM and 0.13 nM, respectively, and their Tops were 47.8, 40.7 and 41.4, respectively. These results indicate that these three mutations had substantially equivalent ADCP activity. Among them, SIRP6-IgG4 had the best activity.

### Example 9. Evaluation of Antitumor Effects of Mutants Described Above in Mice

Each CB-17 SCID mouse (purchased from Vital River) was inoculated with 1 × 10⁷ Daudi cells by intravenous injection in the tail. Then the mice were randomized into groups of 10. The Control group was injected with an antibody-free solvent, i.e., phosphate buffered saline. SIRP6-IgG1 and CV1-IgGl (a fusion protein where CV1 is linked to the Fc of human IgG1) were both administered at a dose of 20 mg/kg. To each group of mice, the drugs were administered twice a week by intraperitoneal injection, and a total of 7 doses were administered. After the administration, the mice were observed daily for survival. Kaplan-Meier survival analysis was performed on each group of mice using Graphpad Prism 7.

Survival curves for each group of mice are shown in FIG. 12. The analysis results show that the median survivals for Control, SIRP6-IgG1 and CV1-IgG1 were 20 days, 52 days and 33 days, respectively. There were still mice survived in the SIRP6- IgG 1 treatment group at the end of the experiment on day 70.

The above results indicate that SIRP6- IgG 1 exerted a stronger in vivo antitumor effect than CV1-IgG 1.

### Example 10. Evaluation of Agglutination Effects of Mutants Described Above on Human Red Cells

There are a large number of red cells in the human blood circulatory system. CD47 is expressed on the surfaces of the red cells. Clinical studies have shown that CD47 antibodies have some hematological toxicity, which leads to anemia. In this example, the agglutination effects of the mutants described above on human red cells were evaluated by cell imaging.

To RPMI-1640 (Gibco, catalog No. 11835-030) was added 10% fetal bovine serum. Human red cells were resuspended in the medium, and then the cell suspension was inoculated into a 6-well cell culture plate at a cell density of 1 million/mL. Then SIRP6-IgG1 or anti-CD47B-Hu-IgG1 was added to each well of cells at a final concentration of 1 µg/mL. After half an hour, the agglutination of red cells was observed with a microscopic imaging system (OLYMPUS, IX53) and recorded.

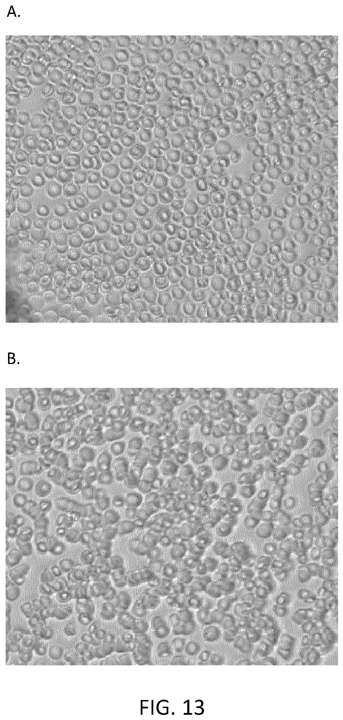

FIGs. A and B in FIG. 13 show the results of the treatment of red cells with SIRP6-IgG1 and that with anti-CD47B-Hu-IgG1, respectively. As shown in the figures, the red cells did not agglutinate after SIRP6- IgG 1 was added, while the red cells notably agglutinated after anti-CD47B-Hu-IgG1 was added.

The results indicate that the SIRP6- IgG 1 of the present invention had superior low toxicity and side effect.

## Claims

1. An SIRPα-Fc fusion protein, wherein the fusion protein comprises an SIRPα D1 domain variant and an immunoglobulin Fc region; the SIRPα D1 domain variant comprises, relative to SEQ ID NO: 1, at least one set of mutations selected from the group consisting of H24N; V27L; I31L; E47Q; E70S; I81V; A84E; R114L; E2T and E1 deletions; L4V and Q5K; A21T and I22V; E65D and S66A; and F103I and K104Q.

2. The fusion protein according to claim 1, wherein the SIRPα D1 domain variant comprises, relative to SEQ ID NO: 1, I31L and at least one additional set of mutations selected from the group consisting of H24N; V27L; E47Q; E70S; I81V; A84E; R114L; E2T and E1 deletions; L4V and Q5K; A21T and I22V; E65D and S66A; and F103I and K104Q.

3. The fusion protein according to any one of claims 1 and 2, wherein the SIRPα D1 domain variant comprises, relative to SEQ ID NO: 1, at least two sets of mutations.

4. The fusion protein according to claim 3, wherein the SIRPα D1 domain variant comprises, relative to SEQ ID NO: 1, the following two sets of mutations: V27L; and I31L.

5. The fusion protein according to any one of claims 1 and 2, wherein the SIRPα D1 domain variant comprises, relative to SEQ ID NO: 1, at least three sets of mutations.

6. The fusion protein according to claim 5, wherein the SIRPα D1 domain variant comprises, relative to SEQ ID NO: 1, the following three sets of mutations: V27L; I31L; and L4V and Q5K.

7. The fusion protein according to any one of claims 1 and 2, wherein the SIRPα D1 domain variant comprises, relative to SEQ ID NO: 1, at least four sets of mutations.

8. The fusion protein according to claim 7, wherein the SIRPα D1 domain variant comprises, relative to SEQ ID NO: 1, the following four sets of mutations: V27L; I31L; L4V and Q5K; and E2T and E1 deletions.

9. The fusion protein according to any one of claims 1 and 2, wherein the SIRPα D1 domain variant comprises, relative to SEQ ID NO: 1, at least five sets of mutations.

10. The fusion protein according to claim 9, wherein the SIRPα D1 domain variant comprises, relative to SEQ ID NO: 1, the following five sets of mutations: V27L; I31L; L4V and Q5K; E2T and E1 deletions; and E65D and S66A;
or the SIRPα D1 domain variant comprises, relative to SEQ ID NO: 1, the following five sets of mutations: V27L; I31L; E65D and S66A; E47Q; and E70S.

11. The fusion protein according to any one of claims 1 and 2, wherein the SIRPα D1 domain variant comprises, relative to SEQ ID NO: 1, at least six sets of mutations.

12. The fusion protein according to claim 11, wherein the SIRPα D1 domain variant comprises, relative to SEQ ID NO: 1, the following six sets of mutations: V27L; I31L; L4V and Q5K; E2T and E1 deletions; E65D and S66A; and E47Q.

13. The fusion protein according to any one of claims 1 and 2, wherein the SIRPα D1 domain variant comprises, relative to SEQ ID NO: 1, at least seven sets of mutations.

14. The fusion protein according to claim 13, wherein the SIRPα D1 domain variant comprises, relative to SEQ ID NO: 1, the following seven sets of mutations: V27L; I31L; L4V and Q5K; E2T and E1 deletions; E65D and S66A; E47Q; and E70S.

15. The fusion protein according to any one of claims 1-14, wherein the SIRPα D1 domain variant further comprises, relative to SEQ ID NO: 1, at least one set of mutations at positions selected from the group consisting of V6; K53; H56; and A66; preferably, two sets of mutations at positions V6 and H56, or three sets of mutations at positions V6; H56; and A66, or four sets of mutations at positions V6; H56; A66; and K53.

16. The fusion protein according to claim 15, wherein the mutations are V6L; K53R; H56N; H56R; H56Q; A66L; A66T or A66G.

17. The fusion protein according to claim 16, wherein the SIRPα D1 domain variant comprises, relative to SEQ ID NO: 1, the following six sets of mutations: V6L; V27L; I31L; E65D and S66A; E47Q; and E70S; or the following seven sets of mutations: V6L; V27L; I31L; E65D and S66A; E47Q; E70S; and H56N; or the following seven sets of mutations: V6L; V27L; I31L; E65D and S66A; E47Q; E70S; and H56R; or the following eight sets of mutations: V6L; V27L; I31L; E65D and S66A; E47Q; E70S; H56R; and A66G; or the following nine sets of mutations: V6L; V27L; I31L; E65D and S66A; E47Q; E70S; H56R; A66G; and K53R.

18. The fusion protein according to any one of claims 1 and 2, wherein the SIRPα D1 domain variant has an amino acid sequence selected from the group consisting of SEQ ID NO: 32 to SEQ ID NO: 39.

19. The fusion protein according to claim 15, wherein the SIRPα D1 domain variant has an amino acid sequence selected from the group consisting of SEQ ID NO: 47 to SEQ ID NO: 51.

20. The fusion protein according to any one of claims 1 and 2, wherein the immunoglobulin Fc region is selected from the group consisting of human IgG1-Fc region, human IgG2-Fc region, human IgG3-Fc region and human IgG4-Fc region.

21. The fusion protein according to claim 15, wherein the immunoglobulin Fc region is selected from the group consisting of human IgG1-Fc region, human IgG2-Fc region, human IgG3-Fc region and human IgG4-Fc region.

22. The fusion protein according to claim 20, wherein the human IgG1-Fc region comprises an amino acid sequence set forth in SEQ ID NO: 2.

23. The fusion protein according to claim 21, wherein the human IgG4-Fc region comprises an amino acid sequence set forth in SEQ ID NO: 40.

24. The fusion protein according to any one of claims 1-23, wherein the SIRPα D1 domain variant is linked to the N-terminus or C-terminus of the immunoglobulin Fc region by a peptide linker.

25. The fusion protein according to claim 24, wherein the peptide linker comprises an amino acid sequence set forth in SEQ ID NO: 3.

26. The fusion protein according to claim 25, wherein the fusion protein has an amino acid sequence selected from the group consisting of SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 41, SEQ ID NO: 42, SEQ ID NO: 43, SEQ ID NO: 44 and SEQ ID NO: 45.

27. A nucleic acid molecule, wherein the nucleic acid molecule encodes the fusion protein according to any one of claims 1-26.

28. The nucleic acid molecule according to claim 27, wherein the nucleic acid molecule has a nucleic acid sequence selected from the group consisting of SEQ ID NO: 16, SEQ ID NO: 18, SEQ ID NO: 20, SEQ ID NO: 22, SEQ ID NO: 24, SEQ ID NO: 26, SEQ ID NO: 28 and SEQ ID NO: 30.

29. An expression vector, wherein the expression vector comprises the nucleic acid molecule according to claim 27 or 28.

30. A host cell, wherein the host cell comprises the expression vector according to claim 29.

31. A method for preparing the fusion protein according to any one of claims 1-26, wherein the method comprises the following steps:
a) culturing the host cell according to claim 30 under expression conditions so the SIRPα-Fc fusion protein is expressed; and
b) isolating and purifying the fusion protein described in step a).

32. A pharmaceutical composition, wherein the pharmaceutical composition comprises an effective amount of the fusion protein according to any one of claims 1-26 and one or more pharmaceutically acceptable carriers, diluents or excipients.

33. Use of the fusion protein according to any one of claims 1-26 or the pharmaceutical composition according to claim 32 for the preparation of a medicament for the treatment of a tumor whose tumor cells express CD47.

34. The use according to claim 33, wherein the tumor is selected from the group consisting of melanoma, kidney cancer, prostate cancer, pancreatic cancer, breast cancer, colon cancer, lung cancer, oesophageal cancer, head and neck squamous cell carcinoma, liver cancer, ovarian cancer, cervical cancer, thyroid cancer, glioblastoma, neuroglioma, leukaemia, lymphoma, myeloma and gastric cancer.

35. A method for treating a tumor, comprising administering to a subject the fusion protein according to any one of claims 1-26 or the pharmaceutical composition according to claim 32, wherein tumor cells of the tumor express CD47.

36. The method according to claim 35, wherein the tumor is selected from the group consisting of melanoma, kidney cancer, prostate cancer, pancreatic cancer, breast cancer, colon cancer, lung cancer, oesophageal cancer, head and neck squamous cell carcinoma, liver cancer, ovarian cancer, cervical cancer, thyroid cancer, glioblastoma, neuroglioma, leukaemia, lymphoma, myeloma and gastric cancer.

37. An immunoconjugate, wherein the immunoconjugate comprises:
(a) the fusion protein according to any one of claims 1-26; and
(b) a conjugated moiety selected from the group consisting of a detectable label, a drug, a toxin, a cytokine, a radionuclide and an enzyme.

38. Use of the immunoconjugate according to claim 37 for the preparation of a pharmaceutical composition for the treatment of a tumor.

39. An SIRPα-Fc mutant protein, wherein the mutant protein comprises an SIRPα D1 protein domain variant; the SIRPα D1 domain variant comprises, relative to SEQ ID NO: 1, at least one or more sets of mutations selected from the group consisting of H24N; V27L; I31L; E47Q; E70S; I81V; A84E; R114L; E2T and E1 deletions; L4V and Q5K; A21T and I22V; E65D and S66A; F103I and K104Q; V6L; K53R; H56N; H56R; H56Q; A66L; and A66T or A66G.

40. The SIRPα mutant protein according to claim 39, wherein the domain variant comprises, relative to SEQ ID NO: 1, the following six sets of mutations: V6L; V27L; I31L; E65D and S66A; E47Q; and E70S; or the following seven sets of mutations: V6L; V27L; I31L; E65D and S66A; E47Q; E70S; and H56N; or the following seven sets of mutations: V6L; V27L; I31L; E65D and S66A; E47Q; E70S; and H56R; or the following eight sets of mutations: V6L; V27L; I31L; E65D and S66A; E47Q; E70S; H56R; and A66G; or the following nine sets of mutations: V6L; V27L; I31L; E65D and S66A; E47Q; E70S; H56R; A66G; and K53R.

41. A nucleic acid molecule, wherein the nucleic acid molecule encodes the mutant protein according to any one of claims 39 and 40 or a fusion protein thereof.

42. An expression vector, wherein the expression vector comprises the nucleic acid molecule according to claim 41.

43. A host cell, wherein the host cell comprises the expression vector according to claim 42.

44. A pharmaceutical composition, wherein the pharmaceutical composition comprises: (i) an effective amount of the mutant protein according to any one of claims 39 and 40 or a fusion protein thereof; and (ii) one or more pharmaceutically acceptable carriers, diluents or excipients.

45. A fusion protein, wherein the fusion protein comprises (a) a first protein element, wherein the first protein element is the mutant protein according to any one of claims 39 and 40, and (b) a second protein element fused with element (a), wherein the second protein element is not derived from SIRPα protein.
